# EUROPEAN PATENT SPECIFICATION

(11) **EP 2 976 126 B1**
(45) Date of publication and mention of the grant of the patent: **03.10.2018**
(21) Application number: 14720014.1
(22) Date of filing: 20.03.2014
(51) Int. Cl.: A61M 37/00

(54) **MICRONEEDLE APPLICATOR COMPRISING A COUNTER ASSEMBLY**
MIKRONADELAUFTRAGSELEMENT MIT EINER ZÄHLERANORDNUNG
APPLICATEUR DE MICRO-AIGUILLES COMPRENANT UN ENSEMBLE COMPTEUR

(30) Priority: 22.03.2013 US 201361804399 P; 22.03.2013 US 201361804387 P; 22.03.2013 US 201361804396 P
(43) Date of publication of application: 27.01.2016
(73) Proprietor: 3M Innovative Properties Company, St. Paul, MN 55133-3427 (US)
(72) Inventor: POON, Yarn Chee, Singapore 768923 (SG); SOON, Boon Yi, Singapore 768923 (SG); JULIUS, David, Singapore 768923 (SG); PAN, Mei-Ling, Singapore 768923 (SG); ZIN, Melvin, Singapore 768923 (SG); LEE, Chi Ying, Singapore 768923 (SG)
(74) Representative: Vossius & Partner Patentanwälte Rechtsanwälte mbB
(86) International application number: PCT/US2014/031307
(87) International publication number: WO 2014/153447

(56) References cited:
- WO-A1-2014/110016
- US-B1- 6 482 186

## Description

### FIELD

The present disclosure generally relates to microneedle applicators and methods for applying a microneedle applicator to skin to treat an area of the skin and/or deliver an active agent to the skin.

### BACKGROUND

Transdermal and topical drug delivery can be used for therapeutic treatment, but the number of molecules that can be effectively delivered using these routes can be limited by the barrier properties of skin. The main barrier to the transport of molecules through the skin is the stratum corneum (the outermost layer of the skin).

A number of different skin treatment methods have been proposed in order to increase the permeability or porosity of the outermost skin layers, such as the stratum corneum, thus enhancing drug delivery through or into those layers. The stratum corneum is a complex structure of compact keratinized cell remnants separated by lipid domains. The stratum corneum is formed of keratinocytes, which make up the majority of epidermal cells, that lose their nuclei and become corneocytes. These dead cells comprise the stratum corneum, which has a thickness of only about 10-30 microns and protects the body from invasion by exogenous substances and the outward migration of endogenous fluids and dissolved molecules. Various skin treatment methods include the use of microneedles, laser ablation, RF ablation, heat ablation, sonophoresis, iontophoresis, or a combination thereof.

Microneedle or micro-pin arrays, also sometimes referred to as microstructured transdermal Systems (MTSs), provide intradermal delivery of active agents, which otherwise would not penetrate the stratum corneum. The sharp microneedle tip is designed to be able to penetrate the stratum corneum layer of the skin, but short enough not to puncture nerve endings, thus reducing or eliminating pain upon insertion. However, the penetration of microneedles to precise levels within the skin tissue and with good reproducibility is often a challenging task. Therefore, unlike the application of traditional patch-based delivery systems, some existing MTSs require the assistance of external energy to ensure efficient and reproducible penetration of microneedles into biological tissue at desired depths. This assistance can be achieved by utilizing an applicator device, which can either be used after positioning the microneedle array on the skin surface, or the applicator device can be integrated with an array of microneedles and, upon activation, can deliver the microneedle array into the skin. The microneedles help to create microchannels in the skin, which in some embodiments, can facilitate delivering an active ingredient. In some constructions, an active ingredient can be applied subsequent to perforating the skin with the microneedles. In some constructions, active component(s) may be coated on the microneedle array and delivered directly through the skin when the stratum corneum is punctured by the microneedles.

Late published document WO 2014110016 (published 17.07.2014, US priority 08.01.2013) relates to an applicator for applying a microneedle device. The applicator comprises a housing having a first open end configured so as to accept the microneedle device, a second end configured as a graspable handle, a driving element contained within the housing, the driving element having a first end configured so as to couple with the microneedle device, an actuation button in mechanical or electrical engagement with the driving element, at least one reciprocating support structure slidably engaged with the housing, wherein the reciprocating support structure has a first position where at least a portion of it extends from the first open end of the housing by a first distance and a second position wherein the portion extends from the first open end of the housing by a second distance, the second distance being less than the first distance.

One advantage of MTS systems over other skin treatment methods is the pain-free mode of delivery. While various designs of microneedle applicators have been shown to produce micro-channels with greater accuracy and reproducibility, there remains a need for microneedle applicators that provide safety assurance and ease-of-use, particularly to an unskilled or naive user (e.g., a consumer), such as to minimize the damage to a user's skin; to limit the maximum amount of force that can be applied (and depth of penetration that can be achieved) via the microneedle array; and to prevent unintentional application, e.g., due to premature or unintentional exposure of the microneedle array.

### SUMMARY

The present invention is defined by the features of the claims.

The present disclosure is generally directed to microneedle applicators comprising a counter assembly that can count the number of times a microneedle array (or microneedle array holder) has been moved to an extended position. That is, the counter assemblies of applicators of the present disclosure can be used to count the number of times a specific microneedle array has been used, and can indicate the number to a user, signifying to the user when to change the microneedle array. Such a counter assembly can automatically perform the count as the applicator is used, which can enhance user compliance and ensure that only simple user instructions are needed to operate the applicator correctly and safely.

The present invention relates to a microneedle applicator comprising a housing having a base and an opening formed in the base, wherein the base of the housing is configured to be positioned on a skin surface; and a microneedle array holder configured to hold a microneedle array. The applicator can further include an actuator movable with respect to the housing between a first position and a second position to cause the microneedle array holder to move, respectively, between (i) a retracted position in which the microneedle array is recessed within the housing such that the microneedle array does not contact the skin surface when the base of the housing is positioned on the skin surface and the microneedle array is coupled to the microneedle array holder, and (ii) an extended position in which at least a portion of the microneedle array is positioned to contact the skin surface when the base of the housing is positioned on the skin surface and the microneedle array is coupled to the microneedle array holder. The applicator can further include a first biasing element configured to bias the actuator in the first position; and a counter assembly configured to count a number of times the microneedle array holder is moved between the retracted position and the extended position.

In addition, in some embodiments, applicators of the present disclosure can provide force dampening when a user attempts to apply a microneedle array onto a substrate that exceeds a threshold (i.e., maximum) application force. Generally, applicators of the present disclosure include a dampening element allows the microneedle array to retract away from the substrate when the threshold application force is met or exceeded. By limiting the amount of force that can be applied via the microneedle array to skin, the applicators can also control the depth of penetration that is achieved with the microneedles.

Furthermore, in some embodiments, applicators of the present disclosure can provide safety and ease-of-use, particularly to an unskilled or naive user (e.g., a consumer), and can minimize premature or unintentional exposure to, or penetration by, the microneedle array. For example, in some embodiments, the applicator can include a cover that can protect a microneedle array located inside the applicator and can limit premature or unintentional exposure of the microneedle array. Such a cover can also be movable with respect to an actuation axis (e.g., to an "off-axis" position), to provide clearance for loading a microneedle array into the applicator and to prevent unintentional application, e.g., due to premature or unintentional exposure of the microneedle array.

Some embodiments of the present disclosure provide applicators that can include one or more of: (i) a cover positioned to limit exposure to a microneedle array coupled to (or located inside) the applicator while allowing the cover to be (re)movable to an off-axis position (e.g., to facilitate microneedle array loading); (ii) means for limiting the amount of force that can be applied to the skin via the microneedle array, and (iii) a counter mechanism for counting (and optionally displaying or indicating) the number of times a microneedle array coupled to the applicator has been used.

Other features and aspects of the present disclosure will become apparent by consideration of the detailed description and accompanying drawings.

### BRIEF DESCRIPTION OF THE DRAWINGS

FIG. 1 is a front perspective view of a microneedle applicator according to one embodiment of the present disclosure.
FIG. 2 is a front exploded perspective view of the microneedle applicator of FIG. 1.
FIG. 3 is front exploded perspective view of the microneedle applicator of FIGS. 1 and 2, the microneedle applicator shown as including an actuator and a microneedle array holder.
FIG. 4A is a rear elevational view of the microneedle applicator of FIGS. 1-3, with portions removed for clarity, the actuator shown in a first position, and the microneedle array holder and the microneedle array shown in a retracted position.
FIG. 4B is a rear elevational view of the microneedle applicator of FIGS. 1-4, with portions removed for clarity, the actuator shown in a second position, and the microneedle array holder and the microneedle array shown in an extended position.
FIG. 5 is a top plan view of a tray comprising a microneedle array.
FIGS. 6A-6E are front elevational views of the microneedle applicator of FIGS. 1-5 that illustrate a process for loading a microneedle array into the microneedle applicator.
FIG. 7A-7C are front cross-sectional views of the microneedle applicator of FIGS. 1-6E that illustrate a process for applying a microneedle array to a substrate, while controlling the force applied to the substrate.
FIG. 8 is a top exploded perspective view of an assembly of the microneedle applicator of FIGS. 1-7C comprising the actuator and the microneedle array holder.
FIG. 9 is an exploded perspective view of a counter assembly of the microneedle applicator of FIGS. 1-8.
FIGS. 10A-10D are side partial cross-sectional views of the microneedle applicator of FIGS. 1-9 that illustrate operation of the counter assembly.
FIG. 11 is a close-up side cross-sectional view of an exemplary microneedle array that can be employed with the microneedle applicator of FIGS. 1-10D, the microneedle array shown with the microneedles pointing upwardly.

### DETAILED DESCRIPTION

Before any embodiments of the present disclosure are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "connected," "supported," and "coupled" and variations thereof are used broadly and encompass both direct and indirect connections, supports, and couplings. It is to be understood that other embodiments may be utilized, and structural or logical changes may be made without departing from the scope of the present disclosure. Furthermore, terms such as "front," "rear," "top," "bottom," and the like are only used to describe elements as they relate to one another, but are in no way meant to recite specific orientations of the apparatus, to indicate or imply necessary or required orientations of the apparatus, or to specify how the invention described herein will be used, mounted, displayed, or positioned in use.

While typically unnecessary or even undesirable in the field of pharmaceuticals or transdermal drug delivery, in some fields and applications, it may be possible or even necessary to reuse the same microneedle array. For example, in cosmetic applications, it may be necessary to treat a desired surface area of skin, such as a contoured surface of a face. Some microneedle designs allow for repeat use, particularly, if repeat use will not negatively affect accurate drug dosing, e.g., in applications where the microneedles are used to perforate skin prior to a topical application of a desired substance. However, most microneedles cannot be repeatedly used to puncture or perforate skin indefinitely, and the microneedles will eventually dull. The number of uses may vary depending on the makeup and configuration of the microneedles.

The applicators of the present disclosure generally provide applicators comprising a counter mechanism or assembly that allow the same microneedle array to be reused, while counting the number of uses to adequately notify a user when a maximum number of uses has been met. At that point, the user can exchange the microneedle array for a fresh, unused array. Some embodiments of the present disclosure employ simple, robust, mechanical counter assemblies that allow some of the same components that drive the actuation of the applicator to serve a dual function of also driving the counter mechanism. Such robust applicators can be relatively inexpensive to manufacture and can allow for counters to be employed in the applicators with a minimal number of additional componentry and without requiring on-board electronics. Such features can be particularly advantageous for consumer-based applications.

Some aspects of the present disclosure can provide microneedle applicators that allow the microneedle array, and particularly, a microneedle array holder configured to hold and move the microneedle array, to retract away from a substrate (e.g., a skin surface) to which the microneedle array is being applied by employing a dampening element. Such a dampening element can include a biasing element that biases the microneedle array holder in an extended position, but which allows the microneedle array holder to be moved to a dampened position against the bias of the biasing element when a threshold (i.e., maximum) application force is applied to the microneedle array. Such an application force ultimately will be 'felt' by the applicator as a result of the skin pressing back on the microneedle array (e.g., in a direction generally normal to the skin surface and/or normal to a first skin-facing side of the microneedle array or microneedle array holder); however, the source of the force comes from a user pressing (e.g., over-pressing) the microneedle applicator onto the skin.

In addition, some aspects of the present disclosure can provide a microneedle applicator comprising a cover that is movable with respect to an actuation axis to provide clearance for loading a microneedle array onto a microneedle array holder. After the microneedle array has been loaded onto the holder, the cover can be returned to its initial position where it can at least partially house the microneedle array prior to actuation (i.e., delivery of the microneedle array to a desired substrate, e.g., a skin surface). The cover can include a base configured to be positioned on a skin surface and an opening formed in the base through which the microneedle array can pass, e.g., along an "actuation axis" when the applicator is actuated to deliver the microneedle array (i.e., to puncture or perforate skin with the microneedle array). In some embodiments, the actuation axis can be oriented substantially normal to a surface to which the microneedle array is to be applied, and can also be substantially normal to the base of the cover (or a tangent thereof). The cover can also be movable to a position (i.e., an "off-axis position") where the actuation axis no longer passes through the opening in the cover, and the microneedle array holder is more exposed, e.g., to facilitate loading a microneedle array onto the holder.

Microneedle applicators of the present disclosure are particularly suitable for consumer-based applications where the applicator will be operated primarily by naive or unskilled users, as opposed to medical practitioners. Such microneedle applicators are intuitively shaped and designed to convey to a user how the applicator should be operated. For example, in some embodiments, the applicator can be elongated along the actuation axis (i.e., the actuation axis can also be, or be parallel to, the longitudinal axis of the applicator), clearly conveying to a user that the applicator is configured to stamp a microneedle array toward the skin surface, as opposed to using the applicator to scrape or drag a microneedle array along the skin surface. Simple stamping or pressing of the microneedle array into a skin surface can be much less invasive and damaging to skin than abrading or otherwise scraping along an outer surface of the skin. The counter assembly also provides an unskilled user with a count of the number of times a microneedle array has been used to avoid unsafe or ineffective microneedle array application, and to signify to a user when to change the microneedle array.

While some existing applicators and microneedle arrays are designed to be left in place on the skin, continuing to puncture the skin and/or deliver an active agent, the applicators of the present disclosure are designed to allow the microneedles to puncture the skin up to a desired force and to a desired depth, but are generally not designed to be left on the skin for an extended treatment period.

Applicators of the present disclosure may be useful when applied to the skin as a "pretreatment" step, that is, when applied to the skin to disrupt the stratum corneum layer of skin and then removed. The disrupted area of skin may then be useful for allowing enhanced delivery of a topical composition (e.g., a solution, a cream, a lotion, a gel, an ointment, or the like) or patch comprising an active agent that is applied to the disrupted area. Applicators of the present disclosure may also be useful when the microneedles are provided with a dried coating comprising an active agent that dissolves from the microneedles after they are inserted into the skin. As a result, applicators of the present disclosure may have utility for enhancing delivery of molecules to the skin, such as in dermatological treatments, vaccine delivery, or in enhancing immune response of vaccine adjuvants. Furthermore, in some embodiments, the active agent may be applied to the skin (e.g., in the form of a solution that is swabbed onto the skin surface, or as a cream, lotion, gel, ointment, or the like, that is rubbed into the skin surface) prior to applying the microneedles of the applicators of the present disclosure.

When a patch is applied to the treated or disrupted site, the patch can be provided in a variety of forms and can include a drug reservoir comprising an active agent for delivery to the treated site. Any transdermal patch suitable for the continuous transdermal delivery of a therapeutically effective amount of an appropriate medicament may be used. Suitable transdermal patches include gelled or liquid reservoirs, such as in U.S. Patent No. 4,834,979 (Gale), so-called "reservoir" patches; patches containing matrix reservoirs attached to the skin by an adjacent adhesive layer, such as in U.S. Patent No. 6,004,578 (Lee et al.), so-called "matrix" patches; and patches containing pressure-sensitive adhesive (PSA) reservoirs, such as in U.S. Patent Nos. 6,365,178 (Venkateshwaran et al.), 6,024,976 (Miranda et al.), 4,751,087 (Wick) and 6,149,935 (Chiang et al.), so-called "drug-in-adhesive" patches. In some embodiments, the drug reservoir can be provided in
the form of a matrix layer containing drug, the matrix layer being adhered to a skin-contact adhesive of the patch. Such a matrix may be an adhesive layer. Alternatively, the matrix layer may be non-adhesive or weakly adhesive and rely upon the surrounding rim of skin-contact adhesive on an adhesive patch to secure the patch in place and keep the drug reservoir in contact with the skin surface.

In another embodiment, the drug reservoir can be provided in the form of solid particles embedded on the surface or within the skin-contact adhesive of the patch. In particular, these particles may be hydrophilic, so that contact with aqueous fluid exposed at the surface of the treated skin will cause them to dissolve or disintegrate, thus releasing drug into the skin.

In another embodiment, the drug reservoir can be provided within the skin-contact adhesive of the patch. The drug may be mixed with the skin-contact adhesive prior to forming the patch or it may be applied to the skin-contact adhesive of the patch in a separate process step. Examples of suitable methods for applying drug to an adhesive layer may be found in U.S. Patent Application Publication No. 2003/054025 (Cantor et al.) and U.S. Patent No. 5,688,523 (Garbe et al.).

The length of time between (i) treatment of the skin with microneedles to increase permeability and (ii) placement of the active agent in contact with the treated skin area may vary. In some embodiments, this length of time can be kept to a minimum in order to avoid any possibility of the skin barrier reforming through a healing process. The minimum length of time can be generally governed by the time it takes to remove the applicators of the present disclosure from the skin and apply the active agent, for example, by swapping on a solution, rubbing in a cream or lotion, remove the liner of a patch and applying its adhesive over the treated site (e.g., if a patch is being employed), etc. This time may be less than about 1 minute, less than about 30 seconds, less than about 10 seconds, or less than about 5 seconds. There is no reason, however, that this time cannot be extended to many minutes or hours if so desired. It is generally known that the length of time that the skin will remain increasingly permeable after treatment depends on the type of treatment and whether the skin is occluded or not after treatment. In some instances, increased permeability can be maintained for up to several days as long as the treated site remains occluded and even in the absence of occlusion the skin may have increased permeability for up to several hours. Thus, if it presented some convenience or clinical benefit, one could treat the site and delay delivery of an active agent/ingredient by wearing some type of dressing over the treated site until such time as one desired to begin delivery of the active agent, at which time the active agent could be applied to the treated skin.

In discussing the applicators of the present disclosure, the term "downward," and variations thereof, is sometimes used to describe the direction in which microneedles are pressed into skin, and "upward" to describe the opposite direction. However, those of skill in the art will understand that the applicators can be used where the microneedles are pressed into skin at an angle to the direction of the earth's gravity, or even in a direction contrary to that of the earth's gravity, and these terms are only used for simplicity and clarity to describe relative directions.

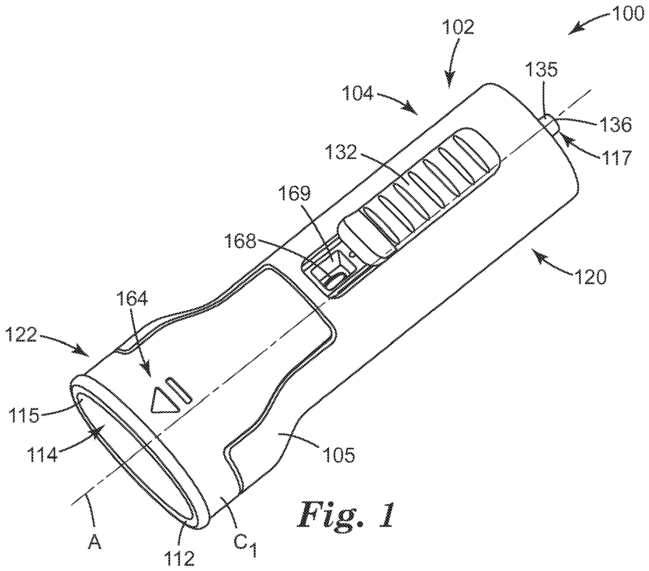

FIGS. 1-10D illustrate a microneedle applicator 100 according to one embodiment of the present disclosure. The applicator 100 can include a housing 102; an actuator 104; a microneedle array holder 106 configured to hold and carry a microneedle array 107 comprising a plurality of microneedles 108; a counter assembly or mechanism 110; a first biasing element 111; a second biasing element 113; and an ejector 117 configured to eject a microneedle array 107 from the applicator 100 (e.g., by decoupling the microneedle array 107 from the holder 106).

In some embodiments, the microneedles 108 can be configured to treat skin (i.e., create small holes or perforations or micropores in the skin) and/or deliver an active agent via skin, particularly, mammalian skin, and particularly, transdermally. Various microneedles that can be employed in applicators and methods of the present disclosure are described in greater detail below.

The term "transdermally," and variations thereof, is generally used to refer to any type of delivery of an active ingredient that crosses any portion of skin. That is, transdermally can generally include systemic delivery (i.e., where the active ingredient is transported across, or substantially through, the dermis such that the active ingredient is delivered into the bloodstream), as well as intradermal delivery (i.e., where the active ingredient is transported partially through the dermis, e.g., across the outer layer (stratum corneum) of the skin, where the active ingredient is delivered into the skin, e.g., for treating psoriasis or for local anesthetic delivery). That is, transdermal delivery as used herein includes delivery of an active ingredient that is transported across at least a portion of skin (but not necessarily all of the layers of skin), rather than merely being topically applied to an outer layer of the skin.

The "microneedle array" 107 can include the microneedles 108 and any supporting structure or substrate used to support the microneedles 108 and/or to couple the microneedle array 107 to other structures or components of the applicator 100, such as the microneedle array holder 106. For example, in some embodiments, the "microneedle array" 107 can include a substrate (or carrier) 109 from which the microneedles 108 protrude, as well as additional layers or carriers. In the embodiment illustrated in FIGS. 1-10D, the microneedles 108 are formed in or directly coupled to the substrate 109, and the substrate 109 is coupled to a base or support 101. However, it should be understood that additional layers can be employed in the microneedle array 107, and other suitable configurations are possible. For example, in some embodiments, the microneedles 108 can be formed directly into the base 101, such that the additional substrate or carrier 109 is not necessary. Also, in such embodiments, the microneedles 108 could extend across the entire area of a first side 116 of the microneedle array 107.

The microneedle array 107 (e.g., the substrate 109 and/or the base 101) can include the first side 116 comprising the microneedles 108 and a second side 118 opposite the first side 116. The first side 116 can include a first major surface (e.g., defined by the substrate 109 in the illustrated embodiment) from which the microneedles 108 protrude. The first side 116 can be oriented toward the base 112 of the housing 102 (i.e., positioned to face the skin surface 50). That is, a microneedle array 107 can be coupled to the microneedle array holder 106 such that the second side 118 faces the microneedle array holder 106, and the first side 116 is oriented toward the base 112 of the housing 102, i.e., positioned to face the skin surface 50, or be "skin-facing."

The housing 102, the actuator 104, the microneedle array holder 106 and/or the microneedle array 107 (e.g., the substrate 109) can be formed of a variety of materials, including but not limited to, thermoset plastics (e.g., acetal resin available under the trade designation DELRIN® DuPont Corporation, Wilmington, DE; other suitable thermoset plastics, or combinations thereof), thermoformable plastics (e.g., polyethylene, polypropylene, other suitable thermoformable plastics, or combinations thereof), or metals (e.g., stainless steel, aluminum, other suitable metals, or combinations thereof), or combinations thereof.

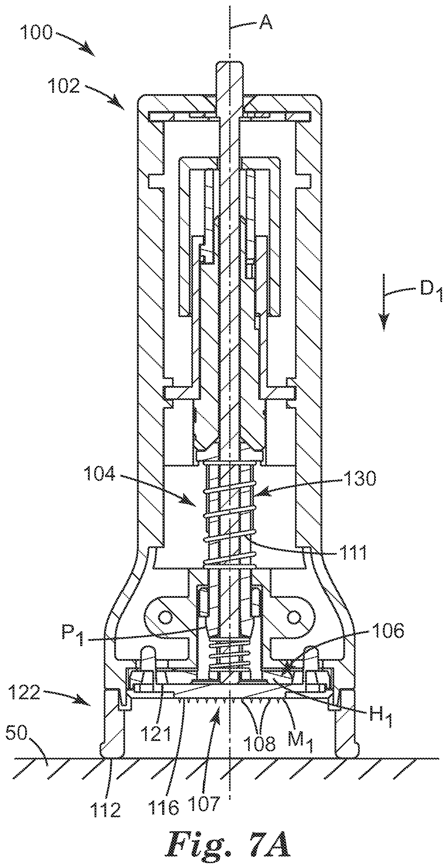

In some embodiments, the housing 102 can include a base 112, which can be generally planar and configured to be positioned toward a skin surface 50 (see, e.g., FIGS. 7A-7C). The base 112 can be configured to touch the skin surface 50 during application; however, the base 112 is generally not configured to remain coupled to the skin 50, and does not include an adhesive. That is, generally, the base 112 is a non-adhesive surface. The housing 102 can further include or define a cavity (or chamber, or pocket, or recess, etc.) 114. As shown, the base 112 can define an opening 115 that opens into the cavity 114. The housing 102, and particularly, the cavity 114 (or a portion thereof) can be configured to house at least a portion of the microneedle array holder 106 and the microneedle array 107 (e.g., when coupled to the holder 106), i.e., prior to application of the microneedles 108 to the skin 50.

In some embodiments, an elastomeric annular cap can be coupled to the base 112 surrounding the opening 115, which can enhance the connection and/or friction between the housing 102 and the skin 50 when the applicator 100 is positioned on the skin 50 for use.

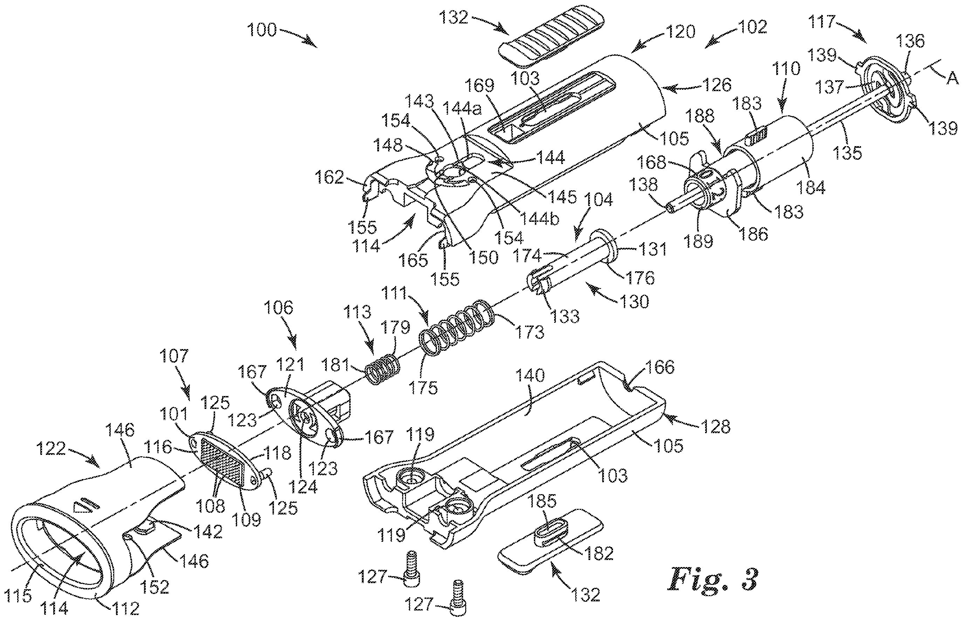

The microneedle array holder 106 can be located within the housing 102 and can be configured to hold a microneedle array 107 and to stamp the microneedles 108 into a substrate of interest (e.g., skin). As shown in FIG. 3, the microneedle array holder 106 can include a first (or bottom) side 121 that can be configured to be positioned toward a skin surface, i.e., skin-facing, and which can be configured to receive the microneedle array 107. By way of example only, the microneedle array holder 106 is shown as having two apertures 123 that open at the first side 121 and which are each dimensioned to receive an upwardly-extending projection 125 from a microneedle array 107. By way of example only, the projections 125 and the apertures 123 can be sized and configured to allow each projection 125 to pass through an aperture 123 and snap onto a backside of the holder 106 via a snap-fit-type engagement. In addition, as shown in FIG. 3, the holder 106 can include one or more projections or flanges 167 that can be configured to be coupled to an outer surface or edge of the microneedle array 107. By way of example, the projections 167 of the illustrated embodiment are configured to be coupled to an outer edge of the base 101 of the microneedle array 107 in a snap-fit-type engagement.

The snap-fit engagement between the microneedle array 107 and the holder 106 of the illustrated embodiment can allow for facile engagement between a microneedle array 107 and the holder 106; however, the above-described types of coupling or engagement are shown by way of example only, and it should be understood that a microneedle array 107 can be coupled (i.e., removably coupled) to the holder 106 by a variety of coupling means, including, but not limited to, press-fit or friction-fit engagement, other types of snap-fit engagement, magnets, hook-and-loop fasteners, adhesives, cohesives, clamps, heat sealing, stitches, staples, screws, nails, rivets, brads, crimps, detents, other suitable coupling means, or combinations thereof.

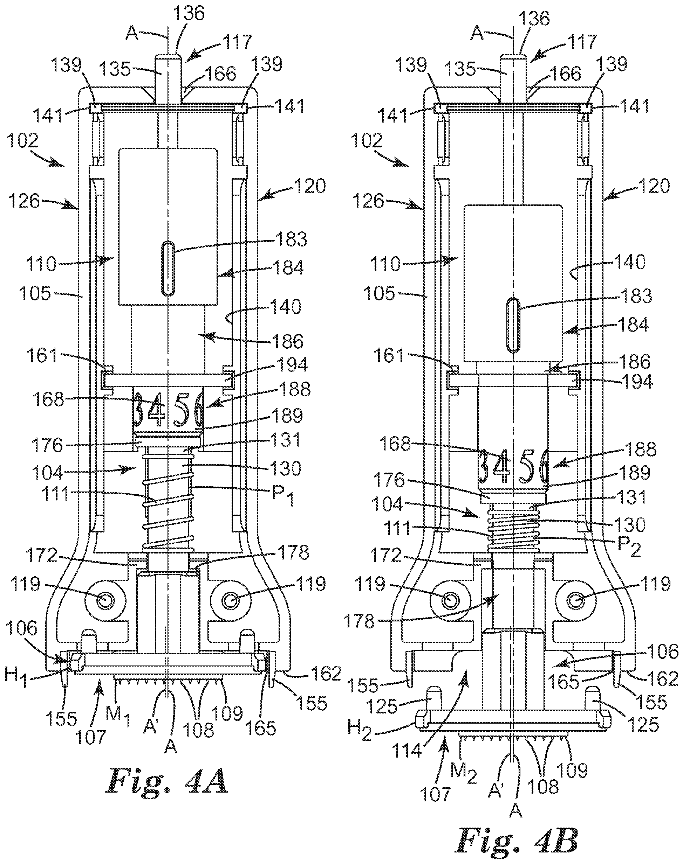

The ejector (or "ejector assembly") 117 can include an elongated pin or shaft 135 that can extend generally along (or generally parallel to) a longitudinal axis A and can include a portion that is accessible from the exterior of the housing 102 (see FIGS. 1 and 4A-4B). By way of example only, the ejector pin 135 can include a first end 136 that extends beyond a top of the housing 102 (e.g., via an opening 166 formed in a top wall or portion of the housing 102), and a second end 138 that can be dimensioned to pass through a bore 124 in the microneedle array holder 106. The ejector pin 135 can be movable between a first position in which the ejector pin 135 (e.g., the second end 138 thereof) does not extend past the first side 121 of the holder 106 and/or does not interfere with the microneedle array 107, to a second position in which the ejector pin 135 (e.g., the second end 138 thereof) does extend past the first side 12 and/or does interfere with the microneedle array 107. The ejector pin 135 can be moved relative to the housing 102, the holder 106, the microneedle array 107 and optionally other components of the applicator 100 with sufficient force to decouple the microneedle array 107 from the microneedle array holder 106, causing the microneedle array 107 to be ejected from the applicator 100, allowing the microneedle array 107 to be discarded and/or replaced by another microneedle array 107.

By way of example only, the ejector 117 can include a biasing element (or a resilient element) 137 configured to bias the ejector pin 135 in its first position, while allowing the ejector pin 135 to be moved against the bias of the biasing element 137 to move the ejector pin 135 to its second position. By way of example only, the ejector biasing element 137 is in the form of a flat spring; however, any suitable biasing element, such as those described below, can be employed. The ejector 117, or a portion thereof (e.g., one or more projections or flanges 139) can be configured to be coupled to the housing 102 so as to fix the ejector 117 with respect to the housing 102 and the longitudinal axis A. By way of example only, in the illustrated embodiment, two (e.g., diametrically-opposed) projections 139 on the ejector 117 are each received within a channel 141 formed in an inner surface 140 of the housing 102 (see FIGS. 4A and 4B).

As shown in FIGS. 3, 4A and 4B, in some embodiments, the ejector pin 135 can serve to align at least some of the other components or elements of the applicator 100, e.g., to align such elements centrally with respect to the housing 102. Such alignment can also facilitate coupling various elements together and can facilitate their collective and/or interactive movement. Such elements can include the counter assembly 110, at least a portion of the actuator 104, and the microneedle array holder 106. As a result, the ejector pin 135 can be centered with respect to the housing 102 and can be aligned along the longitudinal axis A of the applicator 100.

The actuator 104 can include a plunger (or shaft, or piston) 130, as well as any other components that are configured to facilitate movement of the plunger 130. For example, as shown, in some embodiments, the actuator 104 can include one or more buttons 132 or other manually engageable portions or elements that are coupled to the plunger 130 but at least partially reside on the exterior of the housing 102 to allow a user to manually manipulate and control the plunger 130 when the applicator 100 is assembled. The buttons 132 are illustrated by way of example as being slide buttons. Two simultaneously-operable slide buttons are shown by way of example only and for facile user manipulation.

As shown, the plunger 130 can include a first end 131 that can be coupled to, or can interact with, the buttons 132 (e.g., via the counter assembly 110 or other intervening elements, if employed), and a second end 133 that is coupled to, or can interact with, the microneedle array holder 106. By way of example only, the second end 133 of the plunger 130 is illustrated as being dimensioned to be received within a recess 134 formed in an upper portion of the holder 106. Particularly, the second end 133 is configured to be coupled inside the recess 134 via a snap-fit-type engagement. This type of engagement is illustrated by way of example only, but it should be understood that any of the coupling means described above (with respect to the coupling between the microneedle array holder 106 and the microneedle array 107) can be employed.

In some embodiments, the actuator 104 can further include at least a portion of the counter assembly 110, if employed, of which a portion can interact with or be coupled to the button(s) 132, and another portion can interact with and/or be coupled to the plunger 130 (e.g., the first end 131 thereof). In the illustrated embodiment, when a user slides the buttons 132 downwardly (e.g., toward a skin surface 50) relative to the housing 102 from an initial position to an actuated position, the buttons 132 can cause at least a portion of the counter assembly 110 to also move downwardly relative to the housing 102, which in turn, can cause the plunger 130 to move from a first position P₁ (see FIG. 4A) to a second position P₂ (see FIG. 4B). As a result, the buttons 132, the plunger 130, and any other intervening structures or elements (such as portions of the counter assembly 110, if employed) can collectively be referred to as the "actuator" 104. Alternatively, in some embodiments, the plunger 130 can itself be referred to as the "actuator" and the buttons 132, the actuator 130, and any other intervening structures or elements (such as portions of the counter assembly 110, if employed) can collectively be referred to as an "actuator assembly."

The actuator 104 can move as an ensemble in response to user manipulation. Generally, the above-described elements making up the actuator 104 are coupled together and movable together from the first position P₁ to the second position P₂. As a result, the collection of components described above will be referred to in the following description as simply the "actuator" 104, and such description will be understood to apply to any of the above-described components of the actuator 104, as well as the collection of such components. While the elements of the actuator 104 are generally fixed with respect to one another for effective energy transfer and movement, it should be understood that, in some embodiments, there may be some give or play between elements of the actuator 104.

The first biasing element 111 can be configured to bias the actuator 104 in the first position P_{1,} such that a user can move the actuator 104 to the second position P₂ against the bias of the first biasing element 111, and the first biasing element 111 can return, or assist in returning, the actuator 104 to the first position P_{1.}

The actuator 104 can be movable with respect to the housing 102 (e.g., against the bias of the first biasing element 111) between the first position P₁ and the second position P₂ to cause the microneedle array holder 106 to move, respectively, between
(i) a first, retracted position H₁ (see, e.g., FIGS. 4A, 6C, 6E, 7A, 10A, and 10D), and
(ii) a second, extended (or "loading" or "treatment") position H₂ (see, e.g., FIGS. 4B, 6D, 7B, and 10B).

The first, retracted position H₁ and the second, extended position H₂ can be spaced a distance from one another along an actuation axis A', such that the microneedle array holder 106 is movable along the actuation axis A', e.g., relative to the housing 102, between the first, retracted position H₁ and the second, extended position H₂. In some embodiments, as shown, the actuation axis A' can be aligned along the longitudinal axis A of the applicator 100 (or of the housing 102), such that the holder 106 is movable along the direction in which the applicator 100 (or the housing 102) is elongated. Such embodiments can provide an intuitive and user-friendly design. In some embodiments, the actuation axis A' may not be exactly aligned along the longitudinal axis A, but the actuation axis A' can be substantially parallel to the longitudinal axis A. However, the actuation axis A' need not be aligned along or substantially parallel to the longitudinal axis A.

The actuation axis A' can generally be oriented substantially normal with respect to the skin surface 50 (and the first side 121 of the holder 106, as well as the first side 116 of the microneedle array 107 when coupled to the holder 106), but this need not be the case. Rather, in some embodiments, the actuation axis A' can be arcuate or define otherwise nonlinear path(s), etc. The actuation axis A' simply refers to movement between the first, retracted position H₁ and the second, extended position H₂.

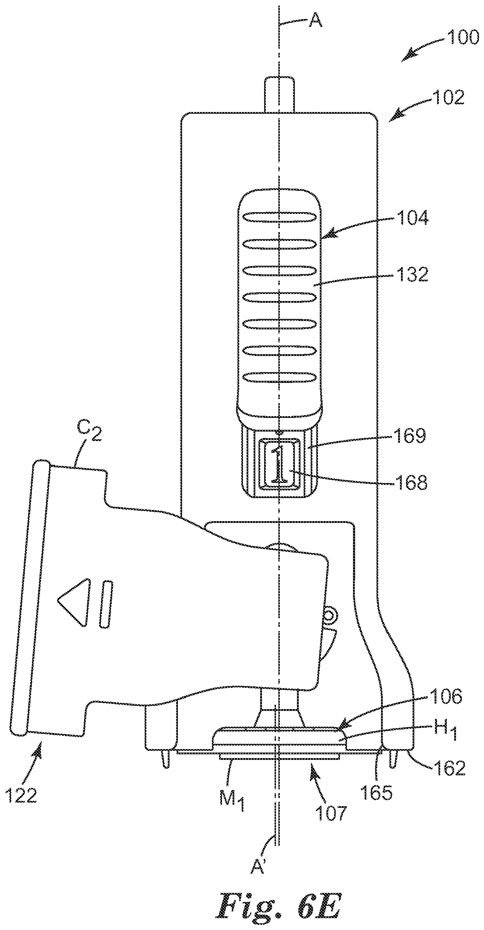

When the microneedle array holder 106 is in the first, retracted position H₁, the holder 106 can be recessed within the housing 102, such that the holder 106 (and the microneedle array 107, when coupled to the holder 106) does not extend beyond the base 112 of the housing 102. That is, when the microneedle array holder 106 is in the first, retracted position H₁ and a microneedle array 107 is coupled to the holder 106, the microneedle array 107 can also be in a first, retracted position M₁ (see, e.g., FIGS. 6E and 7A), e.g., in which the microneedle array 107 is recessed within the housing 102 such that the microneedle array 107 does not contact (or is not positioned to contact) the skin surface 50 when the base 112 of the housing 102 is positioned on the skin surface 50. The microneedle array 107 can be housed within the cavity 114 and can be recessed with respect to the base 112 in its retracted position M₁.

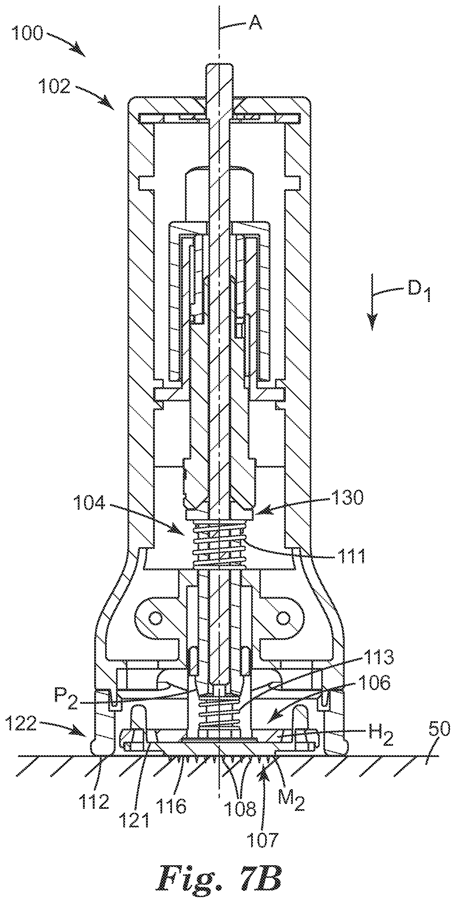

When the microneedle array holder 106 is in the second, extended position H₂ and a microneedle array 107 is coupled to the holder 106, the microneedle array 107 can also be in a second, extended position M₂ (see, e.g., FIG. 7B), e.g., in which at least a portion of the microneedle array 107 is positioned to contact the skin surface 50 when the base 112 of the housing 102 is positioned on the skin surface 50.

When the microneedle array holder 106 and the microneedle array 107 are in their respective second positions H₂ and M₂, at least a portion of the microneedle array 107 (and, potentially, a portion of the microneedle array holder 106) can extend beyond the base 112 of the housing 102. However, this need not be the case, and in some embodiments, it can be preferred for this not to be the case. Rather, in some embodiments, the microneedles 108 can be positioned close enough to the base 112 of the housing 102 (while still being recessed within the housing 102 and without extending beyond the base 112), such that when the base 112 is pressed onto the skin surface 50, the skin 50 is caused to deform or dome up through the opening 115 and into the cavity 114 to a position where the skin 50 is contacted by the microneedles 108. This configuration can be preferred, e.g., in the case of an unskilled user, where it is desirable for the microneedles 108 never to extend beyond the base 112 of the housing 102 in such a way that the unskilled user could potentially use the applicator 100 to scrape the microneedles 108 along the skin surface 50.

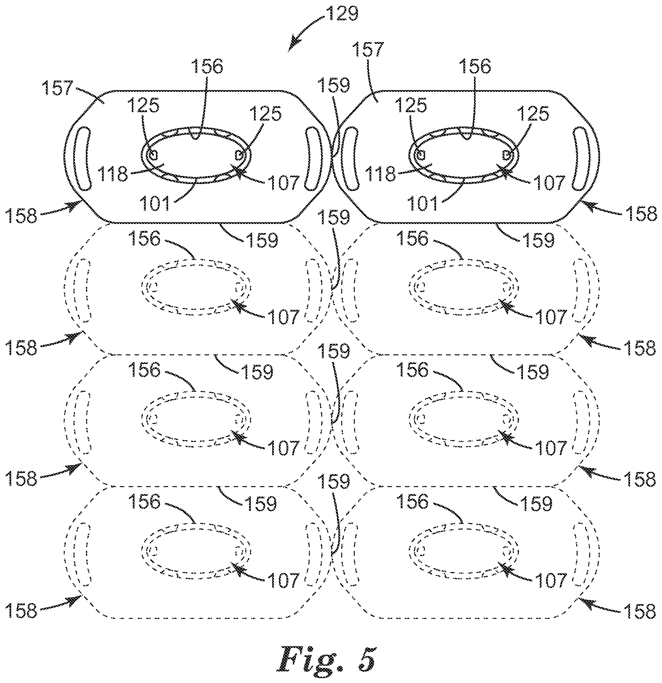

The second, extended position H₂ can also be used to load a microneedle array 107 onto the holder 106, e.g., by picking up a microneedle array 107 from a loading tray 129, as shown in FIG. 5. In some embodiments, this can be facilitated by allowing the microneedle array holder 106, or a portion thereof (e.g., the first side 121 thereof) to extend beyond the base 112 of the housing 102. In such embodiments, the second, extended position H₂ can be located relative to the base 112 of the housing 102 at a position that allows for loading a microneedle array 107, as well as for perforating the skin surface 50 with the microneedles 108.

However, in some embodiments, such as in embodiments in which it is generally preferred that the microneedles 108 not extend beyond the base 112 of the housing 102, at least a portion of the housing 102 can be movable relative to the remainder of the housing 102 and/or removable from the remainder of the housing 102 in order to expose the microneedle array holder 106. Such a movable and/or removable portion of the housing 102 can allow the microneedle array holder 106 to easily extend beyond a portion of the housing 102, e.g., to load a microneedle array 107. The portion of the housing 102 that can be movable and/or removable can then be replaced, or returned to its original position, after the microneedle array 107 has been loaded.

That is, as shown in FIGS. 1-3 and 6A-6E, in some embodiments, the housing 102 can be formed of more than one portion, and at least one of the plurality of portions can be movable with respect to, and/or removable from, other portion(s) in order to open or access an interior of the microneedle applicator 100. In some embodiments, that same (re)movable portion can define the base 112, the opening 115 and a portion of the cavity 114. As shown in the embodiment of FIGS. 1-10D, in some embodiments, the housing 102 can include (i) a first portion 120 configured to house most of the components of the applicator 100, such as the actuator 104, the microneedle array holder 106, the counter assembly 110 (if employed), and the first biasing element 111; and (ii) a second portion 122 that defines the base 112 and the opening 115 formed therein that is movable and/or removable to a position (i.e., an "off-axis position") where the actuation axis A' no longer passes through the opening 115, and the microneedle array holder 106 is exposed. Such an "off-axis" position can facilitate loading a microneedle array 107 onto the holder 106. In some embodiments, the second portion 122 can be referred to as a "cover," and the first portion 120 of the housing 102 can be referred to as the "housing" 102. For simplicity, this nomenclature will be used below to describe the illustrated embodiment.

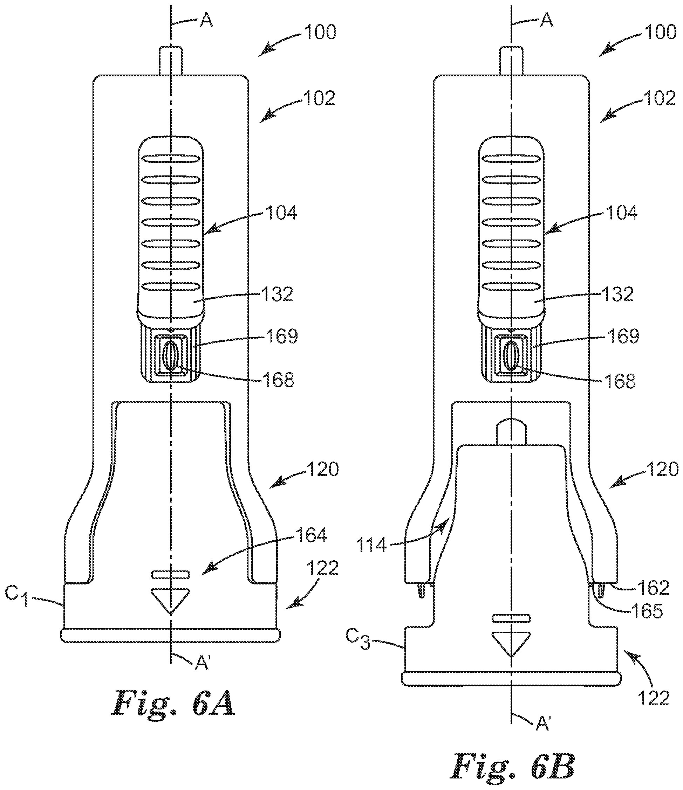

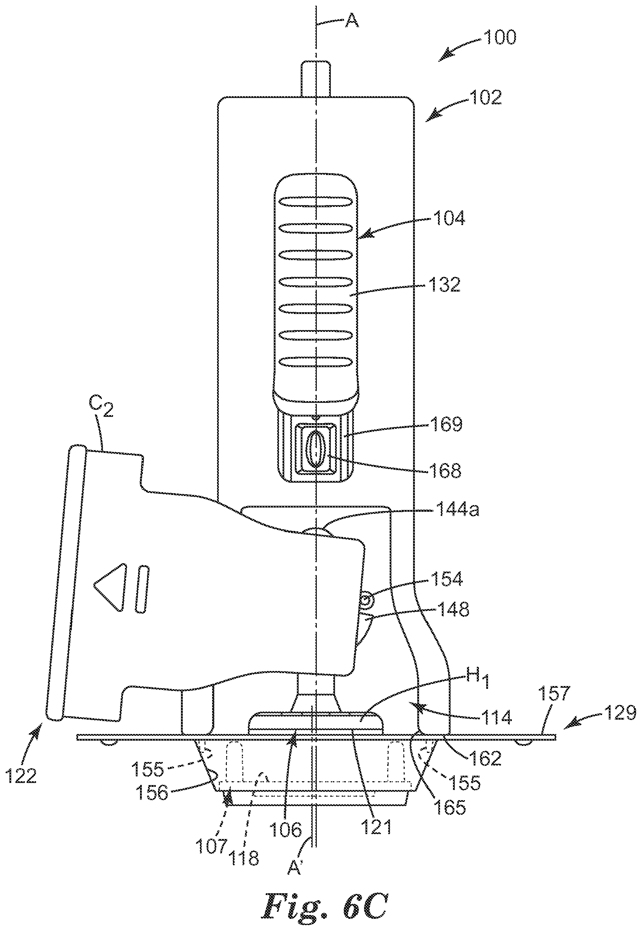

The cover 122 can define the base 112 and the opening 115, can be configured to be positioned on the skin surface 50, and can be configured to be easily grasped and manipulated (e.g., by hand). The cover 122 can be movable, relative to the housing 102 (and the microneedle array holder 106, as well as the other components, including the actuator 104 and the counter assembly 110) between
(i) a first (or "use" or "treatment") position C₁ (see, e.g., FIGS. 1 and 6A) in which the actuation axis A' along which the microneedle array holder 106 is movable passes through (or is aligned with) the opening 115 in the base 112 of the cover 122, and
(ii) a second (or "loading") position C₂ (see, e.g., FIGS. 6C-6E) in which the cover 122 is located in an "off-axis" position relative to the longitudinal axis A of the housing 102 and/or relative to the actuation axis A' of the microneedle array holder 106 in which the longitudinal axis A and/or the actuation axis A' does not pass through (or is not aligned with) the opening 115 in the base 112 of the cover 122.

The term "off-axis" generally refers to a position in which the axis of interest (e.g., the longitudinal axis A and/or the actuation axis A') does not pass through opening 115, such that when the microneedle array holder 106 is moved between the retracted position H₁ and the extended position H₂, there is no interaction with the cover 122. Said another way, the microneedle array holder 106 (and the microneedle array 107 when coupled to the holder 106) does not enter into, or is not located in, the portion of the cavity 114 defined by the cover 122 when the cover 122 is in the "off-axis" second position C₂.

In some embodiments, as shown in the illustrated embodiment, the cover 122 can be further movable between the first position C₁, the second position C₂, and a third position C₃ (see FIG. 6B) spaced a distance along the longitudinal axis A and/or the actuation axis A' from the first position C₁. That is, in some embodiments, before the cover 122 is moved (e.g., swung, slid, etc.) from the first position C₁ to the "off-axis" second position C₂ (or vice versa) the cover 122 can first be moved (e.g., slid) to the intermediate third position C₃, e.g., to provide clearance for the cover 122 to be moved to the "off-axis" second position C₂. In such embodiments, when the cover 122 is in the first position C₁, the microneedle applicator 100 can be considered to be in an "assembled" configuration, and when the cover 122 is in the third position C₃, the microneedle applicator 100 can be considered to be in a "disassembled" configuration, even though both the first position C₁ and third position C₃ could be considered to be "on-axis." As a result, the third position C₃ is generally not an "off-axis" position, and the actuation axis A' (and the longitudinal axis A) can still pass through the opening 115 when the cover 122 is in the third position C₃; however, this need not be the case. Furthermore, in some embodiments, the third position C₃ can be employed to facilitate movement of the cover 122 between the first position C₁ and the second position C₂, and is located intermediately of the first position C₁ and the second position C₂, such that the cover 122 moves through the third position C₃ when moved between the first position C₁ and the second position C₂.

As shown, in some embodiments, the cover 122 can include one or more indicators 164 that can include directional cues or instructions to clearly indicate to a user (i.e., an unskilled user) how to move the cover 122. By way of example only, in the illustrated embodiment, the cover 122 includes a directional arrow pointing downwardly to indicate to a user that the cover 122 can be slid downwardly e.g., to the third position C₃. Once the cover 122 is in the third position C₃, another indicator or directional cue can be exposed to indicate how to move the cover 122 to the second position C₂, or the cover 122 can be configured to be obviously movable (e.g., slidable and/or pivotable) at this point, so that a user will readily discern how the cover 122 can be moved to an "off-axis" position to expose the microneedle array holder 106.

As mentioned above, in some embodiments, the cover 122 can be removable from the housing 102; however, in some embodiments, the cover 122 can remain coupled to the housing 102 when the cover 122 is in the first position C₁, the second position C₂, and the third position C₃. In embodiments in which the cover 122 is removable from the housing 102, the cover 122 can be in the "off-axis" second position C₂ when the cover 122 is removed or decoupled from the housing 102.

In some embodiments, the cover 122 and the housing 102 can each be formed of more than one part or portion. For example, as shown in FIG. 3, in some embodiments, the housing 102 can be formed of a top portion 126 and a bottom portion 128. In some embodiments, the inner surface 140 of the housing 102, and particularly, the inner surface 140 of the top portion 126 can be configured to receive (e.g., can include various recesses for receiving) a majority of the components of the applicator 100. The top portion 126 and the bottom portion 128 of the housing 102 can be coupled together by any of the coupling means described above, as well as by a variety of other permanent or removable coupling means, including, but not limited to, heat sealing, stitches, staples, screws, nails, rivets, brads, crimps, welding (e.g., sonic (e.g., ultrasonic) and/or thermal welding), any thermal bonding technique (e.g., heat and/or pressure applied to one or both of the components to be coupled), other suitable coupling means, or combinations thereof. By way of example only, in the illustrated embodiment, the top portion 126 and the bottom portion 128 are configured to snap together, and further include two threaded apertures 119 for receiving two screws 127.

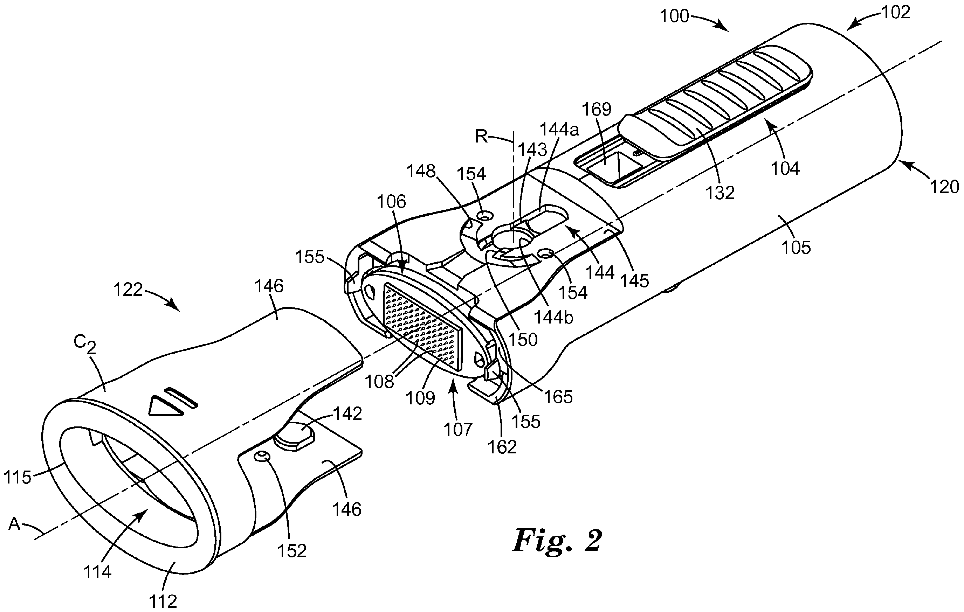

As shown in FIGS. 2 and 3, at least one of the top portion 126 and the bottom portion 128 of the housing 102 (e.g., an outer surface 145 thereof) can include one or more engagement features configured to engage features of the cover 122, and optionally, further configured to allow relative movement between the cover 122 and the housing 102 to allow the cover 122 to be moved between the first position C₁, the second position C₂, and in some embodiments, the third position C₃. In the illustrated embodiment, the cover 122 includes two arms 146, and each arm 146 includes engagement features configured to engage with engagement features formed in the outer surface 145 of the top portion 126 and the bottom portion 128 of the housing 102. By way of example only, each arm 146 of the cover 122 includes one or more first projections 142 dimensioned to be received in one or more recesses 144 formed in the outer surface 145 of the housing 102. Since each arm 146 of the cover 122 and each of the top portion 126 and the bottom portion 128 of the housing 102 include the same engagement features, one side will be described for simplicity, but it should be understood that the same description can apply to the other side as well.

A first recess 144a formed in the outer surface 145 of the housing 102 can be elongated and dimensioned to match the shape of the first projection 142 on the cover 122, which is also elongated in shape, such that when the first projection 142 is received in the first recess 144a, the first projection 142 is seated in the first recess 144a, and the cover 122 is not able to be moved (e.g., rotated) to the "off-axis" second position C₂. The second recess 144b can be round and/or dimensioned to allow the first projection 142 to be rotated therein about a rotational axis R (see FIG. 2) that is oriented substantially orthogonally with respect to the longitudinal axis A of the applicator 100 (or the housing 102) and/or with respect to the actuation axis A' of the holder 106. In addition, the rotational axis R can be considered to be oriented substantially perpendicularly with respect to a direction or line that is normal to the skin surface 50, or substantially parallel with respect to the skin surface 50 (or a tangent thereof). The first recess 144a and the second recess 144b can be connected via a first slot 143 that can be dimensioned to receive the first projection 142 and allow the first projection 142 to be moved (e.g., slid longitudinally) between the first recess 144a and the second recess 144b. The recesses 144 can also be described as forming portions of the first slot 143, i.e., defining detent positions, or stops, along the first slot 143. The first recess 144a corresponds to the first position C₁ of the cover 122, and the second recess 144b corresponds to the third position C₃ of the cover 122.

As mentioned above, the second recess 144b can allow for rotation of the first projection 142 therein, which can also allow for rotation of the cover 122 relative to the housing 102, such that when the first projection 142 is located in the second recess 144b, the cover 122 can swivel or pivot relative to the housing 102, i.e., to be moved to the second position C₂. In the illustrated embodiment, by way of example only, the cover 122 can be moved to one of two second positions C₂, i.e., the cover 122 can be moved to either side relative to the housing 102 in order to move to the second position C₂. However, in some embodiments, only one second position C₂ is available to the cover 122, or the cover 122 is moved to the second position C₂ by being at least partially decoupled from the housing 102.

The engagement features on the housing 102 can further include an arcuate slot 148 that can be connected to the first slot 143 and/or the second recess 144b by a second slot 150. The arcuate slot 148 is positioned symmetrically about the first slot 143 and the recesses 144, which allows the cover 122 to be moved to either side of the housing 102 to move to a second position C₂. The arcuate slot 148 and the second slot 150 can be dimensioned to receive a second projection 152 on the cover 122 that is spaced a distance (e.g., a longitudinal distance) from the first projection 142. By way of example only, the first projection 142 is larger than the second projection 152, and the second projection round in cross-section and is domed. The first projection 142 and the second projection 152 can be located relative to one another such that when the first projection 142 is positioned in the first recess 144a, the second projection 152 is located in the second recess 144b. As the cover 122 is slid downwardly relative to the housing 102, the first projection 142 is moved into the second recess 144b, and the second projection 152 is moved through the second slot 150, into the arcuate slot 148. The first projection 142 can then be rotated in the second recess 144b, and the second projection 152 can be moved in (e.g., slid along) the arcuate slot 148 to either side, thus, causing the cover 122 to move to one of two second positions C₂.

In some embodiments, as shown, the engagement features in the outer surface 145 of the housing 102 can further include one or more third recesses 154, which can each be spaced a distance from an end of the arcuate slot 148 and can define detent positions, or stops, for the second projection 152. Two third recesses 154 are shown by way of example only, each third recess 154 being spaced a short distance from an end of the arcuate slot 148. The third recess(es) 154 can allow the cover 122 to be stopped or held in either of the two second positions C₂. That is, the second projection 152, along with the third recess(es) 154, can be used to create or define detents or stops that correspond to discrete second positions C₂ for the cover 122.

It should be understood that the above-described specific arrangement of engagement features between the housing 102 and the cover 122 is only one possible embodiment of engagement between the cover 122 and the housing 102 that allows for movement (and even discrete positions) between the cover 122 and the housing 102. In addition, the illustrated embodiment allows for decoupling of the cover 122 and the housing 102, because the engagement features on the cover 122 (e.g. the first projection 142 and/or the second projection 152) can be configured to engage the engagement features on the housing 102 (e.g., the first and second recesses 144a, 144b and the third recess 154) in a snap-fit-type engagement, e.g., by providing some flex in arms 146 of the cover 122. Other removable coupling means, such as those described above can also be employed to allow the cover 122 and the housing 102 to be decoupled from one another (and recoupled back together when desired).

The above description and accompanying illustrations describe and illustrate the engagement features on the cover 122 as being the "projections" and engagement features on the housing 102 as being the "recesses." However, it should be understood that this need not be the case, and that the above relative coupling and movement between the cover 122 and the housing 102 can also be achieved with projections provided on the housing 102 and recesses provided on the cover 122. Alternatively, a combination of projections and recesses on each of the cover 122 and the housing 102 can be employed.

With reference to FIGS. 1-2 and 6A-6E, a process for loading a microneedle array 107 into the applicator 100 by moving the cover 122 and the housing 102 relative to one another will now be described. FIGS. 1 and 6A illustrate the applicator 100 in an assembled configuration, with the cover 122 in the first position C₁. In FIG. 6A, the applicator 100 is empty, i.e., the applicator 100 does not yet include a microneedle array 107. As shown in FIG. 6B, the applicator 100 can be changed to a diassembled configuration and the cover 122 can be moved to the third position C₃, spaced a longitudinal (e.g., "on-axis") distance from the first position C₁. As shown in FIG. 2 and described above, movement to the third position C₃ can occur as a result of the first projection 142 on each cover arm 146 moving from its corresponding first recess 144a to its second recess 144b on the housing 102, as well as the second projection 152 on the cover arm 146 moving from the corresponding second recess 144b through the second slot 150 to the arcuate slot 148. In the third position C₃, i.e., when the first projection 142 is positioned in the second recess 144b, the cover 122 can be free to rotate or pivot about the rotational axis R, e.g., to one of a plurality of second positions C₂. That is, the cover 122 can be slidable along the longitudinal axis A of the housing 102 (or the applicator 100) to move between the first position C₁ and the third position C₃, and the cover 122 can be rotatable about the rotational axis R (see FIG. 2) that is oriented substantially perpendicularly with respect to the longitudinal axis A to move between the third position C₃ and the second position C₂.

As further shown in FIG. 6B (see also FIG. 2), as the cover 122 is moved to the third position C₃, a base 162 of the housing 102 (or a base 162 of the first portion 120 of the housing 102) is at least partially exposed. The base 162 of the housing 102 defines an opening 165 formed therein and provides access into the cavity 114 within the housing 102. The microneedle array holder 106, and particularly, the first side 121 thereof, is also at least partially exposed when the cover 122 is moved to the third position C₃. The base 162 can also be configured to be positioned toward the skin surface 50 (i.e., skin-facing), but this need not be the case and depends on the overall configuration of the applicator 100.

As shown in FIG. 6C, the cover 122 can then be moved (e.g., from the third position C₃) to one side of the housing 102 to one of two second positions C₂. As shown in FIG. 2 and described above, movement to a second position C₂ can occur as a result of the first projection 142 being rotated about rotational axis R in the second recess 144b, and as a result of moving the second projection 152 along at least a portion of the arcuate slot 148, and optionally, with enough force to move into one of the third recesses 154. As also shown in FIG. 6C, the base 162 of the housing 102 is exposed when the cover 122 is in the second position C₂, whereas, as shown in FIG. 6A, the base 162 of the housing 102 is covered by the cover 122 when the cover 122 is in the first position C₁. The cover 122, or at least a portion thereof, can also be coupled to the base 162 of the housing 102 when the cover 122 is in the first position C₁. As shown in FIG. 6B, the base 162 can be at least partially exposed when the cover 122 is in the third position C₃. The microneedle array holder 106, and particularly, the first side 121 thereof, is exposed when the cover 122 is moved to the second position C₂.

As further shown in FIG. 6C, the microneedle array holder 106 is empty and does not include a microneedle array 107, and the loading tray 129 comprising a microneedle array 107 is temporarily coupled to the housing 102. By way of example only, the housing 102 includes a pair of projections 155 (see also FIGS. 4A and 4B) that extend downwardly from the base 162, and which are configured to be received within a well (or recess, or pouch) 156 of the tray 129 that comprises the microneedle array 107. By way of example only, the projections 155 fit within the well 156 by a friction-fit or press-fit-type engagement to hold the tray 129 adjacent the base 162 of the housing 102 during the loading process. A top surface 157 of the tray 129 can be positioned adjacent the base 162 of the housing 102, and can optionally include an adhesive.

Some embodiments of the present disclosure provide a kit comprising an applicator of the present disclosure and the loading tray 129 comprising one or more microneedle arrays 107. As shown in FIG. 5, in some embodiments, the tray 129 can include a plurality of wells 156, and each well 156 can include a microneedle array 107. The tray 129 can include one continuous tray 129 with a plurality of wells 156, or the tray 129 can include a plurality of segments 158, as shown, that each include one or more wells 156 (and one or more microneedle arrays 107). Each segment 158 can be separated from an adjacent segment 158 by a score line or perforation 159.

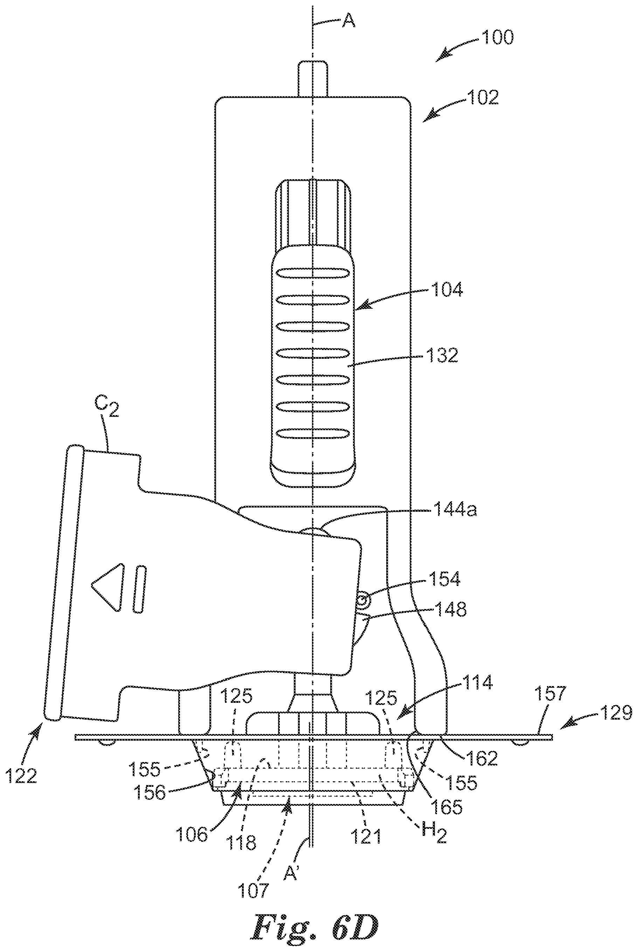

As shown in FIG. 6D, the microneedle array holder 106 is exposed when the cover 122 is in the second position C₂. As such, when the cover 122 is in the second position C₂, the holder 106 can be moved from its retracted position H₁ (see FIG. 6C) to its extended position H₂, e.g., to pick up the microneedle array 107 from the tray 129. The microneedle array holder 106 can be movable through the opening 165 in the base 162 of the housing 102 between its retracted position H₁ and its extended position H₂. As described above, this movement of the holder 106 can be accomplished by a user manipulating the one or more buttons 132, e.g., by sliding the button(s) 132 downwardly relative to the housing 102, which in turn, causes the actuator 104 to move from its first position P₁ to its second position P₂ (see FIGS. 4A and 4B). As described above, in some embodiments, the microneedle array holder 106 can pick up (or load) the microneedle array 107 by pressing the first side 121 of the holder 106 toward the second side 118 of the microneedle array 107 to allow the upwardly-extending projections 125 on the microneedle array 107 to pass through the apertures 123 on the holder 106 and snap onto a backside of the holder 106 (or at least onto a backside of a face of the holder 106). After the holder 106 has picked up the microneedle array 107, and the microneedle array 107 is coupled to the holder 106, the holder 106 can be returned to its retracted position H₁ (and the microneedle array 107) can be positioned in its retracted position M₁, as shown in FIG. 6E. As mentioned above, the first biasing element 111 can bias the actuator 104 in its first position P_{1,} and therefore, the holder 106 can be biased in its retracted position H₁, such that the button(s) 132 merely need to be released after the microneedle array 107 has been coupled to the holder 106 to allow the holder 106 to return to its retracted position H₁. However, a user can also guide the button(s) 132 back to the initial position.

After the microneedle array 107 has been loaded onto the holder 106, and the holder 106 has been moved to its retracted position H₁, the cover 122 can be moved from its second position C₂ back to the third position C₃, as shown in FIG. 6B, and finally, back to the first position C₁, as shown in FIG. 6A. With reference to FIG. 2, in moving from the second position C₂ to the third position C₃, the first projection 142 on the cover 122 can be rotated about rotational axis R in the second recess 144b, and enough force can be applied to overcome the detent between the third recess 154 on the housing 102 and the second projection 152 on the cover 122 to move the second projection 152 into and along the arcuate slot 148 until the second projection 152 is back in line with the second slot 150 (i.e., the third position C₃). Then, in moving from the third position C₃ to the first position C₁, the first projection 142 can be slid in the first slot 143 from the second recess 144b to the first recess 144a, and the second projection 152 can be slid from the arcuate slot 148, through the second slot 150, and into the second recess 144b. The interaction between the first projection 142 and the first recess 144a can also function as a detent or stop to maintain the cover 122 in the first position C₁ until sufficient force is applied to move the first projection 142 out of the first recess 144a.

The above process describes loading a microneedle array 107 into the illustrated embodiment of the applicator 100; however, it should be understood that a very similar process can be employed, with appropriate modifications, to load a microneedle array 107 into any applicator of the present disclosure. For example, in embodiments in which the second position C₂ of the cover 122 is simply a position in which the cover 122 is decoupled from the housing 102 (see FIG. 2), the cover 122 can simply be removed from the housing 102 to expose the microneedle array holder 106 and the base 162 of the housing 102, and the cover 122 can be replaced on the housing 102 after the microneedle array 107 has been loaded.

As described above and illustrated in FIGS. 6A-6E, the extended position H₂ of the holder 106 can be used not only for delivering the microneedles 108 to the skin surface 50, but also for loading a microneedle array 107 onto the holder 106. As a result, the extended position H₂ of the holder 106 can also be referred to as a "loading" position and/or a "treatment" position.

As shown in FIGS. 4A-4B and 6C-6E, in some embodiments, when the cover 122 is removed from the housing 102 or in the second position C₂, the holder 106 can extend beyond the base 162 of the housing 102 when the holder 106 is in its extended position H₂, e.g., to facilitate accessing and picking up a microneedle array 107, and the holder 106 can be at least partially located in the cavity 114 of the housing 102 when in its retracted position H₁.

In some embodiments, the holder 106 does not extend beyond the base 162 of the housing 102 when in its retracted position H₁. In such embodiments, the first side 121 of the holder 106 can be recessed with respect to the base 162 of the housing 102 when the holder 106 is in the retracted position H₁, or the first side 121 can be coplanar or flush with the base 162 of the housing 102. As a result, in some embodiments, the housing 102 can be configured to contain or "house" the microneedle array holder 106 at least when the holder 106 is in the retracted position H₁. In some embodiments, safety can be enhanced by having the holder 106 be sufficiently recessed with respect to the base 162 of the housing 102 when in the retracted position H₁, such that after a microneedle array 107 has been picked up, the microneedle array 107 is also recessed with respect to the base 162. Such a configuration can minimize the likelihood that a user would prematurely or undesirably come into contact with the microneedles 108.

As mentioned above, the cover 122 can be removable from the housing 102, and as such, when the cover 122 is described as being "movable relative to" or "movable with respect to" the housing 102 (or the microneedle array holder 106), it should be understood that this can include being removable from the housing 102 in such a way that the cover 122 can be moved from the "on-axis" first position C₁ to an "off-axis" second position C₂. Furthermore, even in embodiments such as the illustrated embodiment in which the cover 122 is movable relative to the housing 102 (e.g., between the first, second and third positions C₁, C₂ and C₃) while remaining coupled to the housing 102, the cover 122 can also be removable from the housing 102 when the cover 122 is in any of its positions, including the first position C₁, the second position C₂, and the third position C₃.

One advantage of employing a cover 122 that is movable with respect to, or removable from, the housing 102 is that the holder 106 can be exposed to pick up a microneedle array 107 without requiring a user to ever come into contact with the microneedle side of the microneedle array 107. The configuration of the tray 129 and the placement of the microneedle array 107 in a well 156 of the tray 129 (i.e., with the first side 116 of the array 107 facing into the well 156) can also inhibit a user from prematurely or unintentionally coming into contact with the microneedles 108. In addition, after the microneedle array 107 has been picked up by the holder 106, the cover 122 can be moved to the first position C₁ wherein the microneedle array 107 is located within (e.g., entirely within) the cavity 114 (e.g., that is at least partially defined by the cover 122) and is recessed with respect to the base 112, such that the microneedles 108 are not exposed for undesirable or premature puncturing of skin. As such, the (re)movable cover 122 can provide a safety feature to the applicator 100.

As described above, in some embodiments, the cover 122 can be rotatably or pivotally movable with respect to the housing 102, e.g., about the rotational axis R. In addition, the cover 122 can be slidably movable with respect to the housing 102, e.g., as shown in the illustrated embodiment when the cover 122 is moved between the first position C₁ and the third position C₃. As a result, in the illustrated embodiment, the applicator 100 employs rotational (or pivotal) movement and sliding movement between the cover 122 and the housing 102 when the cover 122 is moved between the first position C₁ and the second position C₂. However, it should be understood that in some embodiments, the cover 122 can be slidable with respect to the housing 102 even in moving between the second position C₂ and the third position C₃ (or between the second position C₂ and the first position C₁), and that the slide-and-swivel cover 122 of the illustrated embodiment is shown by way of example only. In some embodiments, a combination of sliding, pivoting and/or other actions can be used to move the cover 122 with respect to the housing 102 between the various positions.

Furthermore, in the illustrated embodiment, the cover 122 is movable between three positions - the first position C₁, the second position C₂ and the third position C₃; however, it should be understood that, in general, the cover 122 is movable between the first position C₁ and an "off-axis" second position C₂, and the intermediate third position C₃ is shown by way of example only. In some embodiments, a plurality of intermediate "third" positions C₃ can be employed.

After the microneedle array 107 has been used (e.g., to its maximum number of applications), the ejector pin 135 can be moved from its first position to its second position, against the bias of the biasing element 137, until the second end 138 of the ejector pin 135 presses against the second side 118 of the microneedle array 107 with sufficient force to decouple the microneedle array 107 and the holder 106 (e.g., to disengage the projections 125 on the microneedle array 107 from the apertures 123 on the holder 106 and to disengage the projections 167 from the base 101 of the microneedle array 107). The cover 122 can be moved to the second position C₂ to facilitate microneedle array loading as well as ejection.

As mentioned above, in some embodiments, the applicator 100 can include a counter assembly 110 that can be configured to count the number of times the microneedle array 107 is applied to skin, and particularly, is configured to count the number of times the microneedle array holder 106 is moved between the retracted position H₁ and the extended position H₂. The counter assembly 110 can include a display 168 (see, e.g., FIGS. 1, 3, 4A-4B, and 6A-6E) that is indicative of this count. For example, in the illustrated embodiment, the display 168 displays an arabic numeral representative of the count. In embodiments employing the counter assembly 110, the housing 102 can include an opening or a window 169 (see, e.g., FIGS. 1-3 and 6A-6E) configured to allow the display 168 to be seen from the exterior of the housing 102 when the applicator 100 is assembled. The top portion 126 of the housing 102 is shown in FIGS. 4A and 4B, with a front of the top portion 126 hidden from view. As a result, the opening 169 is not visible in FIGS. 4A and 4B.

As shown in FIGS. 3, 4A and 4B and described above, the counter assembly 110 can form a portion of, or can be coupled to the actuator 104, such that the counter assembly 110 is also movable (e.g., against the bias of the first biasing element 111) between the first position P₁ and the second position P₂. The counter assembly 110 can be configured to count the movement from the first position P₁ to the second position P₂ as a count, as well as the movement from the second position P₂ to the first position P₁ as an additional count. However, in some embodiments, as in the illustrated embodiment, the counter assembly 110 is configured to count one revolution or one round as a count - i.e., a "count" in the illustrated embodiment represents when the actuator 104 has moved from the first position P₁ to the second position P₂ and back to the first position P₁. The "count" can also be considered to represent the number of times a microneedle array 107 has contacted the skin surface 50, and/or the number of times the microneedle array holder 106 has been moved between the retracted position H₁ and the extended position H₂.

The counter assembly 110 can be employed in embodiments in which it is acceptable for a microneedle array 107 to be applied to the skin surface 50 more than once before being discarded, up to a maximum number of applications. In some embodiments, the applicator 100 can include a locking feature, such that the applicator 100 is inhibited from being used after the maximum number of applications has been reached, e.g., until the microneedle array 107 is discarded (e.g., via the ejector 117) and replaced by a new microneedle array 107. Furthermore, in some embodiments, the counter assembly 110 can be configured to stop at the maximum number of applications until it is triggered to begin recounting by replacing the microneedle array 107.

As shown in FIGS. 4A and 4B and described above, the counter assembly 110 is mechanically coupled to the actuator 104 and the first biasing element 111, such that movement of the actuator 104 (e.g., by manipulating the button(s) 132) and the bias of the first biasing element 111 drive the counter assembly 110. One exemplary counting process is illustrated in FIGS. 6A-6E. As shown in FIG. 6A, the counter display 168 originally displays a "0" when the applicator 100 is empty, representing that the microneedle array 107 has not been used. As shown in FIGS. 6C and 6D, the button(s) 132 can be moved downwardly from an original to an actuated position to move the microneedle array holder 106 to the extended position H₂ to load a microneedle array 107. In the illustrated embodiment, when the button(s) 132 are moved, the display 168 is temporarily not visible, i.e., the opening 169 in the housing 102 is covered by at least one of the buttons 132 when the actuator 104 is in the second position P₂. As the button(s) 132 are allowed to return (e.g., due to the bias of the first biasing element 111), the counter assembly 110 is triggered to increment a count, and as shown in FIG. 6E, the display 168 has incremented a count at this point, and now displays numeral "1."

In some embodiments, it may be preferable that loading the microneedle array 107 does not increment the count, and that the count is not incremented until the microneedle array 107 is actually used after loading. In such embodiments, the first counter display position can display a blank or some other symbol representing that the microneedle array holder 106 is empty, and can increment to a "0" after loading. Then, if the microneedle array holder 106 is moved to its extended position H₂ with the microneedle array 107 coupled to the holder 106, that movement will be counted, and the counter display 168 will increment to a "1."

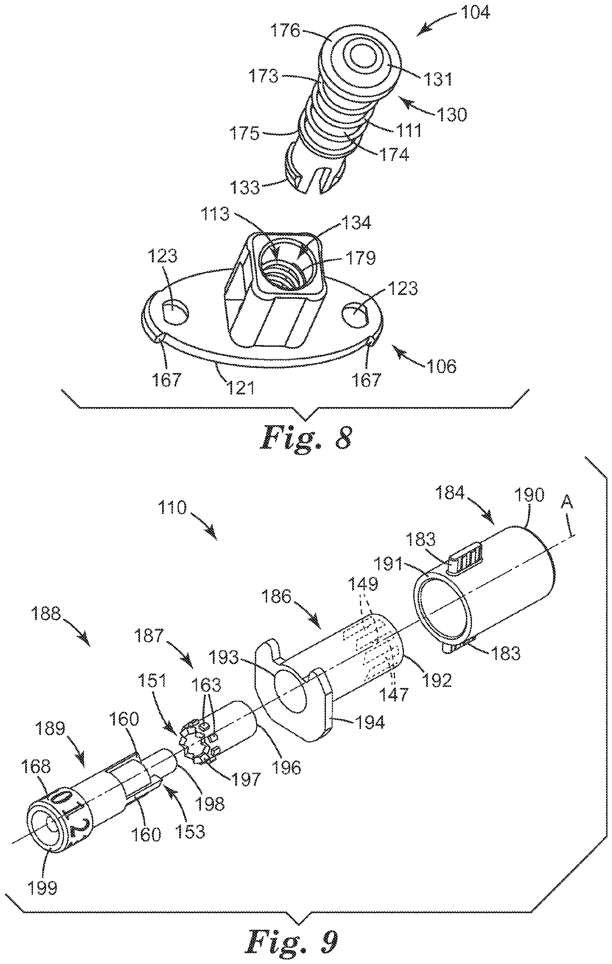

Various counter mechanisms can be employed to achieve this count of the number of uses or applications of the microneedle array 107. One example of a counter assembly that can be employed in applicators of the present disclosure is illustrated in FIGS. 9-10D and described in greater detail below.

The applicator 100 can be configured for safe use, especially by an unskilled user. For example, the movable cover 122 can be employed to enhance the safety of the applicator 100. In some embodiments, the safety of the applicator 100 can be enhanced by limiting the amount of force a user can apply to the skin 50 with the microneedles 108. That is, some embodiments of the applicator 100 can include force dampening configured to limit the threshold application force that can be applied to skin 50 with the applicator 100, and particularly, with the microneedles 108. That is, in some embodiments, the applicator 100 can include a force-dampening mechanism, such that when a user continues to press the applicator 100 (and the microneedles 108) into the skin 50, the applicator 100 is configured to inhibit any force beyond a certain threshold from actually being transferred to the skin 50. One example of a force-dampening mechanism that can be employed is illustrated in FIGS. 7A-7C and 8.

FIGS. 7A-7C illustrate the applicator 100 in cross-section as the applicator 100 is applied to the skin surface 50. FIG. 7A shows the base 112 of the cover 122 (or of the housing 102, in embodiments in which a movable cover 122 is not employed) being applied to the skin surface 50. In FIG. 7A, the actuator 104 is in the first position P₁ with respect to the housing 102 (e.g., with respect to the longitudinal axis A of the housing 102). FIG. 7B shows the actuator 104 being moved against the bias of the first biasing element 111 from the first position P₁ to the second position P₂ to move the holder 106, respectively, from its retracted position H₁ to its extended position H₂, and to also move the microneedle array 107, respectively, from its retracted position M₁ to its extended position M₂, wherein the microneedles 108 are positioned in contact with the skin surface 50 to puncture or perforate the skin surface 50.

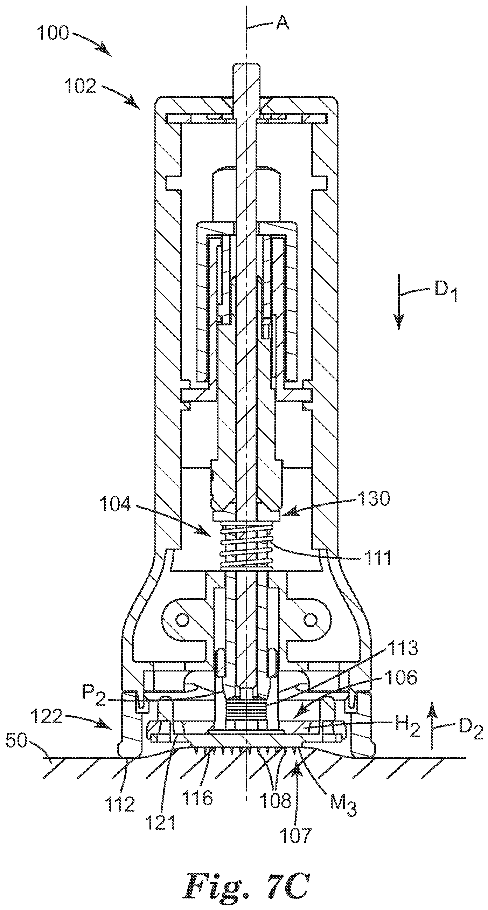

As shown in FIG. 7C, as the applicator 100 (and the microneedles 108) are continued to be pressed into the skin surface 50 with the actuator 104 (e.g., via the button(s) 132) in the second position P₂, the skin 50 will also continue to reciprocally press back with an equal and opposite force on the microneedle array 107. FIG. 7C shows the skin 50 doming or deforming up into the cavity 114 for illustration purposes. If the skin 50 presses back onto the microneedle array 107 (and, in turn, the microneedle array holder 106), and a threshold (e.g., maximum) application force is achieved, the microneedle array 107, will be moved away from the skin surface 50 accordingly, via the microneedle array holder 106, by moving the holder 106 against the bias of the second biasing element 113 from the extended position H₂ to a third, dampened position H₃. Accordingly, the microneedle array 107 can be moved to a dampened position M₃. As a result, the description herein pertaining to the dampened position H₃ of the microneedle array holder 106 can also apply to the dampened position M₃ of the microneedle array 107. The dampened position H₃, M₃ is located intermediately between the retracted position H₁, M₁ and the extended position H₂, M₂. In some embodiments, the second biasing element 113 can be referred to as a "dampening element" or a "force dampening element."

While the dampened position H₃, M₃ is illustrated as one discrete position for simplicity, it should be understood that the "dampened position" H₃, M₃ can refer to any position to which the microneedle array holder 106 (and/or the microneedle array 107) is moved when a threshold application force is achieved that is sufficient to overcome the bias of the second biasing element 113, and can be any of a variety of positions that are located intermediately between the retracted position H₁, M₁ and the extended position H₂, M₂.

A "threshold application force" generally refers to the force required to move the microneedle array holder 106 from its extended position H₂, e.g., to the dampened position H₃. As a result, the second biasing element 113 can be selected to set the desired threshold application force for the applicator 100. In general, the threshold application force is directed substantially perpendicularly with respect to (or in a direction oriented substantially perpendicularly with respect to) the microneedle array 107 (or the microneedle array holder 106), or substantially normal to the skin surface 50. Particularly, this force is applied by the skin 50 onto the first side 116 of the microneedle array 107 (or the first side 121 of the microneedle array holder 106) as a result of the applicator 100 and the microneedle array 107 being pressed on the skin 50.

The phrase "directed substantially perpendicularly with respect to the microneedle array (or holder)" or "in a direction oriented substantially perpendicularly with respect to the microneedle array (or holder)," or variations thereof, generally refers to a motion that is directed generally perpendicularly or normal to a base or major plane of the microneedle array 107 (or the microneedle array holder 106), e.g., normal to a base of the microneedle array 107 (or the microneedle array holder 106). For example, in some embodiments, a direction that is "perpendicular with respect to the microneedle array (or holder)" can be substantially perpendicular to the first side 116 of the microneedle array 107 (or to the first side 121 of the holder 106), and/or to the skin surface 50 to which the microneedle array 107 is being applied. In some embodiments, the microneedle array 107 (and/or the holder 106) can include an arcuate first side 116, or an outer surface that has some curvature or undulations. In such embodiments, a direction that is "perpendicular with respect to the microneedle array (or holder)" would generally refer to a direction that is normal to an outer surface of the microneedle array 107, e.g., normal to a tangent of such an arcuate surface.

As shown in FIG. 7C, the actuator 104 can still be in the second position P₂, and while the actuator 104 is in the second position P₂, the microneedle array holder 106 (and the microneedle array 107) is movable relative to the actuator 104 between the extended position H₂ and the dampened position H₃. In some embodiments, the actuator 104 may not still be held in the second position P₂ when the microneedle array holder 106 is moved to the dampened position H₃, but rather, the microneedle array holder 106 may only be movable to the dampened position H₃ after the actuator 104 has been actuated sufficiently, i.e., after the actuator 104 has been moved to the second position P₂.

As shown, when the microneedle array holder 106 is in the dampened position H₃, the microneedle array holder 106 (and optionally, the microneedle array 107) is located within the cavity 114 of the housing 102 (which can include any portion of the cavity 114 formed by the cover 122, if the movable cover 122 is employed), e.g., such that the microneedle array holder 106 is recessed within the housing 102 and spaced a distance from the base 112 of the housing 102.

As further shown in FIG. 7C, the base 112 is in contact with the skin surface 50, and the actuator 104 is in the second position P₂ when the microneedle array holder 106 is movable to the dampened position H₃. In addition, when the base 112 is held against the skin surface 50, at least a portion of the microneedle array 107 is still positioned to contact the skin surface 50. In some embodiments, the microneedle array holder 106 is only movable to the dampened position H₃ when the actuator 104 is in the second position P₂. Because the microneedle array holder 106 is movable to the dampened position H₃ against the bias of the second biasing element 113, the second biasing element 113 is configured to bias the microneedle array holder 106 in the extended position H₂ such that sufficient skin perforation is achieved by the microneedles 108, but also allows the microneedle array holder 106 to be moved against the bias of the second biasing element 113 when the application force on the microneedle array 107 (e.g., applied to the first side 116 of the microneedle array 107 and the first side 121 of the microneedle array holder 106) meets or exceeds the threshold application force.

In embodiments in which the microneedle array 107 or microneedle array holder 106 are located at least partially beyond the base 112 of the housing 102 when the microneedle array holder 106 is in the extended position H₂, the full extended position H₂ of the microneedle array holder 106 may never fully be reached when applied to the skin surface 50, because the skin surface 50 may immediately press back on the microneedle array holder 106 and cause the microneedle array holder 106 to move to its dampened position H₃.

By way of example only, the first biasing element 111 and the second biasing element 113 are illustrated as being coil springs. As a result, the first biasing element 111 is illustrated as being compressed in FIG. 7B, and the second biasing element 113 is illustrated as being compressed in FIG. 7C. However, it should be understood that a variety of other biasing elements can be employed as the first biasing element 111 or the second biasing element 113, including, but not limited to, deflected beams, leaf springs, flat springs, hinged springs, compression springs (e.g., standard, conical, etc.), torsion springs (e.g., single, double, etc.), extension springs, barrel springs, propellant canisters, resilient or compressible materials (e.g., elastomeric materials, such as natural or synthetic rubbers, other suitable biasing elements, or combinations thereof.

One embodiment of the arrangement of the actuator 104, the first biasing element 111, the microneedle array holder 106, and the second biasing element 113 is illustrated in FIG. 8 by way of example only. Specifically, only a portion of the actuator 104 - the plunger 130 - is shown in FIG. 8 for simplicity and clarity.

As described above, the plunger 130 and the microneedle array holder 106 can be coupled together, such that the microneedle array holder 106 can be moved with movement of the actuator 104. Specifically, in the illustrated embodiment, the recess 134 in the microneedle array holder 106 is dimensioned to receive the second end 133 of the plunger 130, e.g., in a snap-fit-type engagement. As shown in FIGS. 4A and 4B, the first biasing element 111 can be located between an internal wall 172 of the housing 102 and the first end 131 of the plunger 130. Specifically, in some embodiments, as shown, the first biasing element 111 can be configured to receive a shaft 174 (see FIGS. 3 and 8) of the plunger 130. A first end 173 (see FIGS. 3 and 8) of the first biasing element 111 can be configured to abut a flange 176 of the plunger 130, and a second end 175 of the first biasing element 111 can be configured to abut the internal wall 172. As a result, when the actuator 104 is moved from the first position P₁ to the second position P₂, the first biasing element 111 can be compressed between the flange 176 of the plunger 130 and the internal wall 172 of the housing 102, and the actuator 104 (e.g., including the plunger 130) and the microneedle array holder 106 can move relative to the housing 102 together.

As shown in FIGS. 4A and 4B, the internal wall 172 of the housing 102 can define at least a portion (e.g., an upper portion) of an internal cavity or recess 178 (e.g., within the larger cavity 114 that is at least partially defined by one or more outer walls 105 of the housing 102). The internal cavity 178 can be dimensioned to receive at least a portion (e.g., an upper portion) of the microneedle array holder 106. In some embodiments, as shown in the illustrated embodiment, the internal wall 172 can receive an uppermost position of the microneedle array holder 106 and at least partially defines the retracted position H₁ of the holder 106. For this reason, in the illustrated embodiment, when the microneedle array holder 106 is in the retracted position H₁, the microneedle array holder 106 is not able to be "dampened," and the second biasing element 113 remains in a relaxed or uncompressed state.

As further shown in FIG. 8, the second biasing element 113 can be dimensioned to be received within the recess 134 of the microneedle array holder 106. While the bore 124 (see FIG. 3) of the holder 106 configured to receive the ejector 117 is in communication with and continuous with the recess 134, in the illustrated embodiment, the bore 124 has a smaller cross-sectional area than the recess 134, such that the first side (or wall) 121 of the microneedle array holder 106 can serve as a stop for the second biasing element 113. As a result, a first end 179 of the second biasing element 113 can be configured to abut the second end 133 of the plunger 130 that is located in the recess 134 of the microneedle array holder 106, and a second end 181 (see FIG. 3) of the second biasing element 113 can be configured to abut the microneedle array holder 106 (e.g., an internal wall thereof, a base thereof, and/or a second side of the microneedle array 107 that is opposite the first side 121, etc.).

As a result of the above arrangement, the first biasing element 111 biases the actuator 104 upwardly (e.g., away from the skin surface 50), and the second biasing element 113 biases the microneedle array holder 106 downwardly (e.g., toward the skin surface 50), such that the first and second biasing elements 111 and 113 function to bias elements of the applicator 100 in opposite directions. Said another way, with reference to FIGS. 7A-7C, the actuator 104 can be movable from the first position P₁ to the second position P₂ in a first direction D₁ (i.e., downwardly, toward the skin surface 50, e.g., substantially normal to the skin surface 50), and the microneedle array holder 106 can be movable from the extended position H₂ to the dampened position H₃ in a second direction D₂ (i.e., upwardly, away from the skin surface 50, e.g., substantially normal to the skin surface 50). The first direction D₁ and the second direction D₂ can be different from one another, and in some embodiments, as shown, can be directly opposite one another.

One example of a counter assembly will now be described with reference to FIGS. 9-10D. FIG. 9 shows the counter assembly 110 of the illustrated embodiment, which includes a coupler 184, a guide (or "counter guide") 186, and a counter 188. The counter 188 includes a first portion 187 and a second portion 189. By way of example, the second portion 189 of the counter 188 includes the display (or "indicator") 168. As shown in FIG. 9, the first portion 187 and the second portion 189 of the counter 188 are configured to be coupled together and are dimensioned to be received within the guide 186, and the guide 186 is dimensioned to be received with the coupler 184. As shown in FIG. 9, the counter assembly 110 can be aligned along or parallel to the longitudinal axis A (and/or the actuation axis A').

The coupler 184 serves as a means for coupling the counter assembly 110 (and the other components that forms the actuator 104) to the button(s) 132 on the exterior of the housing 102. As shown in FIG. 3, the housing 102 can include one or more slots or apertures 103 formed through its outer wall(s) 105. Each slot 103 can be dimensioned to receive a projection 182 of a button 132. As further shown in FIG. 3, the projection 182 on the button 132 can include a recess 185 formed therein dimensioned to receive a projection (e.g., a radially-outwardly extending projection) 183 of the coupler 184. As such, the projection 182 on the button 132 can slide in the slot 103 formed in the outer wall 105 of the housing 102 as the actuator 104 is moved between the first position P₁ and the second position P₂. As the projection 182 (and the button 132) slides relative to the housing 102, the coupler 184, with its projection 183 received in the recess 185, also slides relative to the housing 102. Two (e.g., diametrically-opposed) projections 183 on the coupler 184 are illustrated by way of example.

The coupler 184 includes a first closed end 190, and a second open end 191. The guide 186 includes a first open end 192, and a second open end 193. The guide 186 also includes a flange 194, which by way of example is illustrated as being adjacent the second end 193 of the guide 186. The flange 194 can be dimensioned to be received within a channel 161 formed in the inner surface 140 of the housing 102 (see FIGS. 4A and 4B), such that the guide 186 remains fixed, or stationary, relative to the housing 102 and the actuator 104 as the actuator 104 is moved between the first position P₁ and the second position P₂. The flange 194 can also have a non-circular shape to inhibit rotational movement. The first portion 187 of the counter 188 includes a first end 196 dimensioned to be received through the first open end 192 of the guide 186, and a second end 197 dimensioned to reside in the guide 186 and to receive a first end 198 of the second portion 189 of the counter 188. The first end 196 of the first portion 187 is also configured to abut the first closed end 190 of the coupler 184. The second portion 189 of the counter 188 also includes a second end 199 configured to be coupled to the first end 131 of the plunger 130 (see FIGS. 3 and 8). By way of example only, the second end 199 of the second portion 189 of the counter 188 can be open and dimensioned to receive at least a portion of the plunger 130 adjacent its first end 131, e.g., such that the second end 199 of the counter 188 can abut the flange 176 of the plunger 130, as shown in FIGS. 4A and 4B.

As a result of the above exemplary coupling and arrangement of elements, as the button 132 and the coupler 184 are slid relative to the housing 102, the first closed end 190 of the coupler 184 abuts the first portion 187 of the counter 188, and the coupler 184 causes the first portion 187 and the second portion 189 of the counter 188 to be slid in the guide 186, as well as causing the plunger 130 to move, which in turn, moves the microneedle array holder 106, as described above, e.g., against the bias of the first biasing element 111.

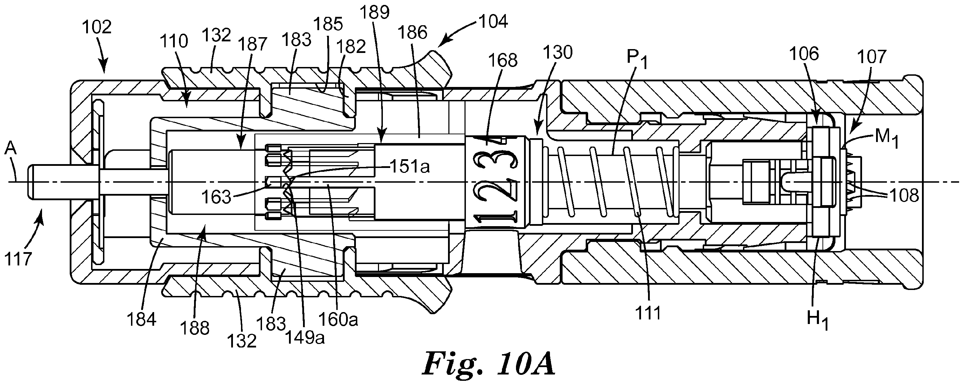

The counter 188 (i.e., the first portion 187 and the second portion 189 thereof) can be slidably movable, or translationally movable (e.g., along the longitudinal axis A) relative to the guide 186 and the housing 102. As illustrated in FIGS. 10A-10D, in some embodiments, the second portion 189 of the counter 188 can further be rotatably movable relative to the guide 186 and the housing 102 (as well as relative to the first portion 187, the ejector 117, the actuator 104 (e.g., the plunger 130), the microneedle array holder 106, and the longitudinal axis A) to increment a count of the number of times the actuator 104 has moved between the first position P₁ and the second position P₂ (or the number of times the actuator 104 has been moved to the second position P₂). Accordingly, the count can be representative of the number of times the microneedle array holder 106 has been moved between the retracted position H₁ and the extended position H₂ (or the number of times the microneedle array holder 106 has been moved to the extended position H₂). As discussed above, the count can therefore be representative of the number of times a microneedle array 107 has been used.

As shown in FIGS. 10A-10D, wherein the guide 186 is shown as being transparent, the first portion 187 of the counter 188 can include a plurality of first engagement features 151, and the second portion 189 of the counter 188 can include a plurality of second engagement features 153 configured to engage the plurality of first engagement features 151 to allow the first portion 187 and the second portion 189 to move or travel together when the actuator 104 is moved between the first position P₁ and the second position P₂. Specifically, in the illustrated embodiment, the first portion 187 and the second portion 189 each include a plurality of interengaging teeth. That is, the first portion 187 includes a plurality of peaks separated by valleys, where the peaks point generally longitudinally downwardly. The second portion 189, and particularly, the second plurality of engagement features 153, can include one or more projections 160 that can be referred to as the "counter projections"). The projection(s) 160 extend generally longitudinally upwardly and include slanted ends that are dimensioned to be received in the valleys of the first engagement features 151 of the first portion 187. The slanted ends of the projections 160 can be referred to as a cam surface, and the plurality of second engagement features 153 can be considered to include one or more cam surfaces. In some embodiments, at least a portion of the plurality of first engagement features 151 along which the slanted ends can slide (e.g., the angled surfaces forming the teeth) can also be referred to as a cam surface.

As shown in FIGS. 9-10D, the guide 186 includes a plurality of projections (e.g., longitudinally extending projections) 147 that are spaced a distance (e.g., a circumferential distance) apart from another about an inner surface (e.g., a cylindrical inner surface) of the guide 186 to define channels (e.g., longitudinally-extending channels) 149 therebetween. The projections 147 extend partially along the length of the guide 186, and their lower ends (i.e., the ends positioned toward the plunger 130) are slanted and configured to allow the slanted ends of the projections 160 of the second portion 189 of the counter 188 to slide or cam therealong. As a result, in some embodiments, the guide 186 can be described as including or defining one or more cam surfaces, and as described in greater detail below, the plurality of second engagement features 153 of the second portion 189 (e.g., the projections 160) of the counter 188 can be configured to cam along the cam surface(s) of the guide 186 to change (i.e., increment) the count. The first portion 187 can include one or more projections (e.g., radially-outwardly-extending projections) 163 that are each dimensioned to be received in and ride along a channel 149 of the guide 186 as the counter assembly 110 is moved. Similarly, the one or more projections 160 of the second portion 189 of the counter 188 are also each dimensioned to be received in and ride along a channel 149 of the guide 186. By way of example only, the first portion 187 of the illustrated embodiment includes one projection 163 per engagement feature 151 and per channel 149 of the guide 186.

Operation of the counter assembly 110 will now be described with reference to FIGS. 10A-10D. FIG. 10A shows the actuator 104 in the first position P_{1,} and the microneedle array holder 106 and the microneedle array 107 in their respective retracted positions H₁ and M₁. When, the actuator 104 is in the first position P₁, one or more projections 163 of the first portion 187 of the counter 188 are each positioned in a channel 149 of the guide 186. That is, in the first position P_{1,} a first engagement feature 151a of the first portion 187 of the counter 188 is abutted against a second engagement feature 153a, i.e., a projection 160a, of the second portion 189 of the counter 188 in a first channel 149a of the guide 186. While the first engagement feature 151a is abutted against the projection 160a, the slanted end of the projection 160a is not fully seated in a valley of the first engagement feature 151a, but their relative positions are maintained by being constrained within the first channel 149a.

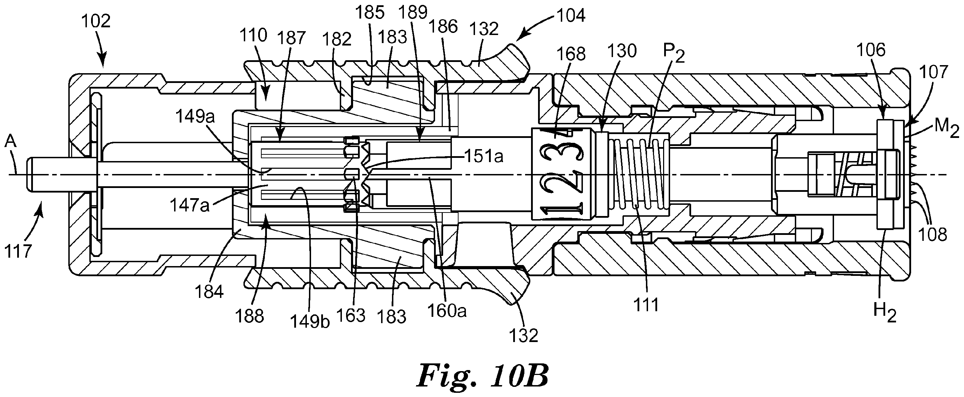

FIG. 10B shows the actuator 104 in the second position P₂ (i.e., against the bias of the first biasing element 111), as well as the microneedle array holder 106 and the microneedle array 107 in their respective extended positions H₂ and M₂. As shown in FIG. 10B, in the second position P₂, the first portion 187 and the second portion 189 of the counter 188 have moved downwardly (e.g., longitudinally) relative to the guide 186, such that the first engagement feature 151a and the second engagement feature 153a, remaining engaged, have moved out of the first channel 149a of the guide 186.

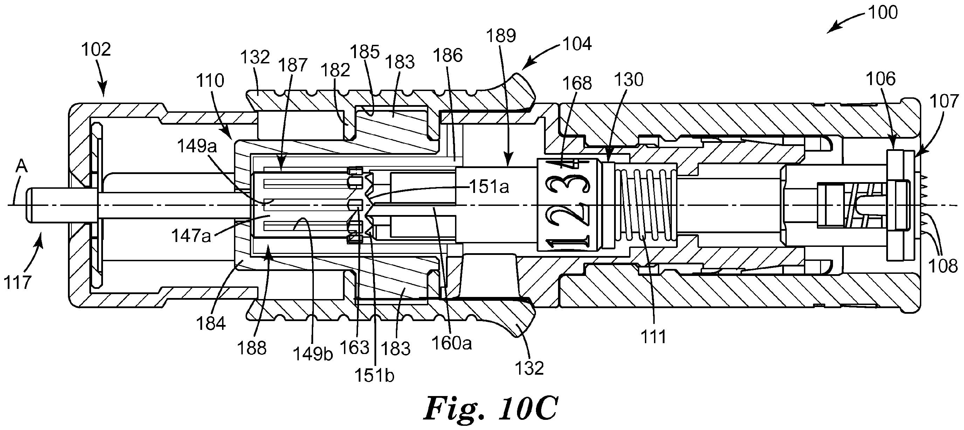

As shown in FIG. 10C, as the button 132 is released or guided back to its initial position, and the actuator 104 is allowed to return to its first position P_{1,} e.g., as a result of the bias of the first biasing element 111, the slanted end of the projection 160a rides along the cam surface provided by the first engagement feature 151a, causing the second portion 189 of the counter 188 to begin to rotate relative to the other elements (e.g., relative to the first portion 187, the guide 186, the coupler 184, the housing 102, etc.) until the projection 160a is fully seated in a valley of the first portion 187. The second portion 189 rotates downwardly relative to the plane of the page of FIGS. 10A-10D.

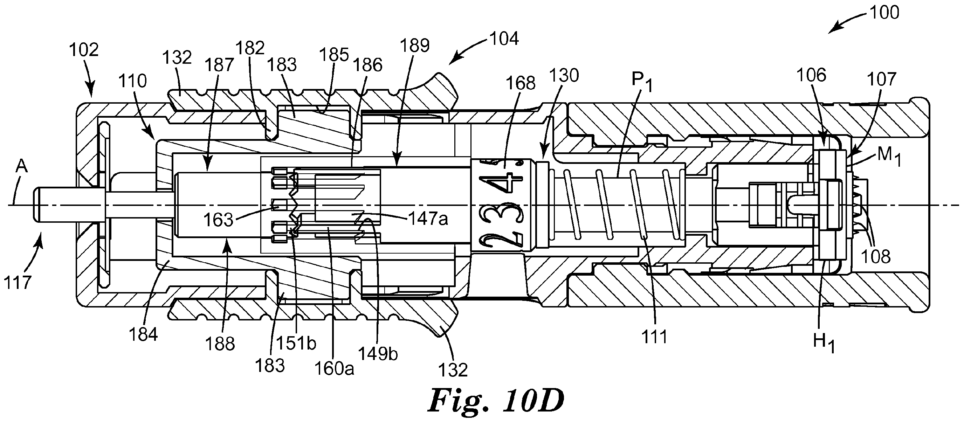

As shown in FIG. 10C, as the actuator 104 continues to move from the second position P₂ to the first position P₁, the projection 160a will be positioned to catch a slanted lower end of a first projection 147a on the guide 186, taking the projection 160a out of engagement with the first engagement feature 151a. The slanted end of the projection 160a on the second portion 189 can then cam along the slanted lower end of the first projection 147a on the guide 186, causing the second portion 189 of the counter 188 to further rotate (i.e., to continue to rotate), forcing the projection 160a into the next channel 149 on the guide 186, i.e., a second channel 149b, where the projection 160a abuts a second engagement feature 151b of the first portion 187 of the counter 188, as shown in FIG. 10D. FIG. 10D shows the actuator 104 returned to the first position P₁, and the microneedle array holder 106 and the microneedle array 107 returned to their retracted positions H₁ and M₁. Movement of the projection 160a into the second channel 149b causes the counter display 168 to increment the count, e.g., that is displayed through the opening 169 in the housing 102. As such, the second portion 189 of the counter 188 can be rotatably movable relative to the opening 169 in the housing 102.

The illustrated embodiment includes discrete movement of the counter 188, such that the portion of the counter 188 that is rotatable (i.e., the second portion 189) is discretely rotatable relative to the housing 102, the longitudinal axis A, and the other elements described above. This discrete movement is due at least in part to the discrete channels 149 in the guide 186 into which the discrete projection(s) 160 can be moved, e.g., sequentially. While continuous or semi-continuous mechanisms can be employed, the discrete motion mechanism of the illustrated embodiment provides a simple, robust, precise and clear count that is not dependent on user variability. In addition, in some embodiments, such as in the illustrated embodiment, based on the construction of the counter assembly 110, the count may not be incremented until the actuator 104 has been fully moved to the second position P₂, or until the microneedle array holder 106 has been fully moved to the extended position H₂. This can be useful in maintaining an accurate count of the number of times a microneedle array 107 has been used, because it does not increment until the microneedle array 107 has been moved sufficiently to contact the skin 50.

Other suitable counting mechanisms can be employed, and the above-described arrangement and interaction of elements is described and illustrated by way of example only. Particularly utility, however, can be found in counter assemblies, such as the counter assembly 110 of the illustrated embodiment, that are mechanically coupled to, and movable with, other elements of the applicator 100. As such, the applicators of the present disclosure can robustly keep track of the number of times a microneedle array 107 has been used without relying on expensive electronic systems. After the maximum number of applications has been achieved for a given microneedle array 107, the microneedle array 107 can be decoupled from the microneedle array holder 106 (e.g., via the ejector 117, e.g., after moving the cover 122 to its second position C₂), and a new microneedle array 107 can be loaded into the applicator 100.

The first biasing element 111 can have multiple functions in the applicator 100. For example, the first biasing element 111 can control the movement of the actuator 104 between its first and second positions P₁ and P₂ (i.e., by returning the actuator 104 to the first position P₁ after a user has moved it to the second position P₂). In turn, this action also controls the movement of the microneedle array holder 106 between its retracted and extended positions H₁ and H₂. As a result, the first biasing element 111 can control loading a microneedle array 107 into the applicator 100, as well as application of the microneedle array 107 onto the skin 50 after the microneedle array 107 has been loaded. In addition, the first biasing element 111 can drive and/or control the operation of the counter assembly 110 for counting the number of uses or applications for a given microneedle array 107.

As mentioned above, in some embodiments, active ingredients or agents (e.g., drugs) can be delivered via the microneedles 108 (e.g., via solid microneedles, as described below). Examples of pharmaceutically active agents (also referred to as "drugs") that can be incorporated into the applicators of the present disclosure are those capable of local or systemic effect when administered to the skin. Some examples include buprenorphine, clonidine, diclofenac, estradiol, granisetron, isosorbide dinitrate, levonorgestrel, lidocaine, methylphenidate, nicotine, nitroglycerine, oxybutynin, rivastigmine, rotigotine, scopolamine, selegiline, testosterone, tulobuterol, and fentanyl, which are commercially available in the form of transdermal devices. Other examples include antiinflammatory drugs, both steroidal (e.g., hydrocortisone, prednisolone, triamcinolone) and nonsteroidal (e.g., naproxen, piroxicam); bacteriostatic agents (e.g., chlorhexidine, hexylresorcinol); antibacterials (e.g., penicillins such as penicillin V, cephalosporins such as cephalexin, erythromycin, tetracycline, gentamycin, sulfathiazole, nitrofurantoin, and quinolones such as norfloxacin, flumequine, and ibafloxacin); antiprotazoals (e.g., metronidazole); antifungals (e.g., nystatin); coronary vasodilators; calcium channel blockers (e.g., nifedipine, diltiazem); bronchodilators (e.g., theophylline, pirbuterol, salmeterol, isoproterenol); enzyme inhibitors such as collagenase inhibitors, protease inhibitors, acetylcholinesterase inhibitors (e.g., donepezil), elastase inhibitors, lipoxygenase inhibitors (e.g., A64077), and angiotensin converting enzyme inhibitors (e.g., captopril, lisinopril); other antihypertensives (e.g., propranolol); leukotriene antagonists (e.g., ICI204,219); anti-ulceratives such as H2 antagonists; steroidal hormones (e.g., progesterone); antivirals and/or immunomodulators (e.g., 1-isobutyl-1H-imidazo[4,5-c]quinolin-4-amine, 1-(2-hydroxy-2-methylpropyl)-1H-imidazo[4,5-c]quinolin-4-amine, N-[4-(4-amino-2-ethyl-1H-imidazo[4,5-c]quinolin-1-yl)butyl]methanesulfonamide, and acyclovir); local anesthetics (e.g., benzocaine, propofol, tetracaine, prilocaine); cardiotonics (e.g., digitalis, digoxin); antitussives (e.g., codeine, dextromethorphan); antihistamines (e.g., diphenhydramine, chlorpheniramine, terfenadine); narcotic analgesics (e.g., morphine, fentanyl citrate, sufentanil, hydromorphone hydrochloride); peptide hormones (e.g., human or animal growth hormones, LHRH, parathyroid hormones); cardioactive products such as atriopeptides; antidiabetic agents (e.g., insulin, exanatide); enzymes (e.g., anti-plaque enzymes, lysozyme, dextranase); antinauseants; anticonvulsants (e.g., carbamazine); immunosuppressives (e.g., cyclosporine); psychotherapeutics (e.g., diazepam); sedatives (e.g., phenobarbital); anticoagulants (e.g., heparin, enoxaparin sodium); analgesics (e.g., acetaminophen); antimigraine agents (e.g., ergotamine, melatonin, sumatriptan, zolmitriptan); antiarrhythmic agents (e.g., flecainide); antiemetics (e.g., metaclopromide, ondansetron, granisetron hydrochloride); anticancer agents (e.g., methotrexate); neurologic agents such as anxiolytic drugs; hemostatics; anti-obesity agents; dopamine agonists (e.g., apomorphine); GnRH agonists (e.g., leuprolide, goserelin, nafarelin); fertility hormones (e.g., hCG, hMG, urofollitropin); interferons (e.g., interferon-alpha, interferon-beta, interferon-gamma, pegylated interferon-alpha); and the like, as well as pharmaceutically acceptable salts and esters thereof. The amount of drug that constitutes a therapeutically effective amount can be readily determined by those skilled in the art with due consideration of the particular drug, the particular carrier, and the desired therapeutic effect.

In some embodiments, peptide therapeutic agents (natural, synthetic, or recombinant) can be delivered via the microneedles 108 (e.g., via solid microneedles). Examples of peptide therapeutic agents that can be incorporated into the applicators of the present disclosure include parathyroid hormone (PTH), parathyroid hormone related protein (PTHrP), calcitonin, lysozyme, insulin, insulinotropic analogs, glatiramer acetate, goserelin acetate, somatostatin, octreotide, leuprolide, vasopressin, desmopressin, thymosin alpha-1, atrial natriuretic peptide (ANP), endorphin, vascular endothelial growth factor (VEGF), fibroblast-growth factor (FGF), erythropoietin (EPO), bone morphogenetic proteins (BMPs), epidermal growth factor (EFG), granulocyte colony-stimulating factor (G-CSF), granulocyte macrophage colony stimulating factor (GM-CSF), insulin-like growth factor (IGF), platelet-derived growth factor (PDGF), growth hormone release hormone (GHRH), dornase alfa, tissue plasminogen activator (tPA), urokinase, ANP clearance inhibitors, lutenizing hormone releasing hormone (LHRH), melanocyte stimulating hormones (alpha & beta MSH), pituitary hormones (hGH), adrenocorticotropic hormone (ACTH), human chorionic gonadotropin (hCG), streptokinase, interleukins (e.g. IL-2, IL-4, IL-10, IL-12, IL-15, IL-18), protein C, protein S, angiotensin, angiogenin, endothelins, pentigetide, brain natriuretic peptide (BNP), neuropeptide Y, islet amyloid polypeptide (IAPP), vasoactive intestinal peptide (VIP), hirudin, glucagon, oxytocin, and derivatives of any of the foregoing peptide therapeutic agents.

In some embodiments, drugs that are of a large molecular weight may be delivered transdermally. Increasing molecular weight of a drug typically can cause a decrease in unassisted transdermal delivery. Examples of such large molecules include proteins, peptides, nucleotide sequences, monoclonal antibodies, vaccines, polysaccharides, such as heparin, and antibiotics, such as ceftriaxone. Examples of suitable vaccines include therapeutic cancer vaccines, anthrax vaccine, flu vaccine, Lyme disease vaccine, rabies vaccine, measles vaccine, mumps vaccine, chicken pox vaccine, small pox vaccine, hepatitis vaccine, hepatitis A vaccine, hepatitis B vaccine, hepatitis C vaccine, pertussis vaccine, rubella vaccine, diphtheria vaccine, encephalitis vaccine, Japanese encephalitis vaccine, respiratory syncytial virus vaccine, yellow fever vaccine, recombinant protein vaccine, DNA vaccines, polio vaccine, therapeutic cancer vaccine, herpes vaccine, human papilloma virus vaccine, pneumococcal vaccine, meningitis vaccine, whooping cough vaccine, tetanus vaccine, typhoid fever vaccine, cholera vaccine, tuberculosis vaccine, severe acute respiratory syndrome (SARS) vaccine, HSV-1 vaccine, HSV-2 vaccine, HIV vaccine and combinations thereof. The term "vaccine" thus includes, without limitation, antigens in the forms of proteins, polysaccharides, oligosaccharides, or weakened or killed viruses. Additional examples of suitable vaccines and vaccine adjuvants are described in U.S. Publication No. 2004/0049150 (Dalton et al.).

In another embodiment, small-molecule drugs that are otherwise difficult or impossible to deliver by passive transdermal delivery may be used. Examples of such molecules include salt forms; ionic molecules, such as bisphosphonates, including sodium alendronate or pamedronate; and molecules with physicochemical properties that are not conducive to passive transdermal delivery.

In some embodiments, the microneedles 108 can be solid. In such embodiments, if an active agent is to be delivered to the skin, the active agent can be applied to the microneedles 108 by a variety of techniques, including, but not limited to painting (e.g., brushing), coating, dipping, or the like, or combinations thereof.

Microneedle arrays useful for practicing the present disclosure can have a variety of configurations and features, such as those described in the following patents and patent applications. One embodiment for the microneedle arrays 107 includes the structures disclosed in U.S. Patent Application Publication No. 2005/0261631 (Clarke et al.), which describes microneedles having a truncated tapered shape and a controlled aspect ratio. Another embodiment for the microneedle arrays 107 includes the structures disclosed in U.S. Patent No. 6,091,975 (Daddona et al.), which describes blade-like microprotrusions for piercing the skin. Still another embodiment for the microneedle arrays 107 includes the structures disclosed in U.S. Patent No. 6,312,612 (Sherman et al.), which describes tapered structures having a hollow central channel. Yet still another embodiment for the microneedle arrays 107 includes the structures disclosed in U.S. Patent No. 6,379,324 (Garstein et al.), which describes hollow microneedles having at least one longitudinal blade at the top surface of the tip of the microneedle. A further embodiment for the microneedle arrays 107 includes the structures disclosed in U.S. Patent Application Publication Nos. US2012/0123387(Gonzalez et al.) and US2011/0213335 (Burton et al.), which both describe hollow microneedles. A still further embodiment for the microneedle arrays 107 includes the structures disclosed in U.S. Patent Nos. 6,558,361 (Yeshurun) and 7,648,484 (Yeshurun et al.), which both describe hollow microneedle arrays and methods of manufacturing thereof.

Various embodiments of microneedles that can be employed in the microneedle arrays of the present disclosure are described in PCT Publication No. WO 2012/074576 (Duan et al.), which describes liquid crystalline polymer (LCP) microneedles; and PCT Publication No. WO 2012/122162 (Zhang et al.), which describes a variety of different types and compositions of microneedles that can be employed in the microneedles of the present disclosure.

In some embodiments, the microneedle material can be (or include) silicon, glass, or a metal such as stainless steel, titanium, or nickel titanium alloy. In some embodiments, the microneedle material can be (or include) a polymeric material, preferably a medical grade polymeric material. Exemplary types of medical grade polymeric materials include polycarbonate, liquid crystalline polymer (LCP), polyether ether ketone (PEEK), cyclic olefin copolymer (COC), polybutylene terephthalate (PBT). Preferred types of medical grade polymeric materials include polycarbonate and LCP.

In some embodiments, the microneedle material can be (or include) a biodegradable polymeric material, preferably a medical grade biodegradable polymeric material. Exemplary types of medical grade biodegradable materials include polylactic acid (PLA), polyglycolic acid (PGA), PGA and PLA copolymer, polyester-amide polymer (PEA).

A microneedle or the plurality of microneedles in a microneedle array useful for practicing the present disclosure can have a variety of shapes that are capable of piercing the stratum corneum. In some embodiments, one or more of the plurality of microneedles can have a square pyramidal shape, triangular pyramidal shape, stepped pyramidal shape, conical shape, microblade shape, or the shape of a hypodermic needle. In some embodiments, one or more of the plurality of microneedles can have a square pyramidal shape. In some embodiments, one or more of the plurality of microneedles can have a triangular pyramidal shape. In some embodiments, one or more of the plurality of microneedles can have a stepped pyramidal shape. In some embodiments, one or more of the plurality of microneedles can have a conical shape. In some embodiments, one or more of the plurality of microneedles can have a microblade shape. In some embodiments, one or more of the plurality of microneedles can have the shape of a hypodermic needle. The shape can be symmetric or asymmetric. The shape can be truncated (for example, the plurality of microneedles can have a truncated pyramid shape or truncated cone shape). In a preferred embodiment, the plurality of microneedles in a microneedle array each have a square pyramidal shape.

In some embodiments, the plurality of microneedles in a microneedle array are solid microneedles (that is, the microneedles are solid throughout). In some embodiments, the plurality of solid microneedles in a solid microneedle array can have a square pyramidal shape, triangular pyramidal shape, stepped pyramidal shape, conical shape, or microblade shape. In a preferred embodiment, the plurality of solid microneedles in a solid microneedle array each have a square pyramidal shape.

In some embodiments, the plurality of microneedles in a microneedle array are hollow microneedles (that is, the microneedles contain a hollow bore through the microneedle). The hollow bore can be from the base of the microneedle to the tip of the microneedle or the bore can be from the base of the microneedle to a position offset from the tip of the microneedle. In some embodiments, one or more of the plurality of hollow microneedles in a hollow microneedle array can have a conical shape, cylindrical shape, square pyramidal shape, triangular pyramidal shape, or the shape of a hypodermic needle.

In some embodiments, one or more of the plurality of hollow microneedles in a hollow microneedle array can have a conical shape. In some embodiments, one or more of the plurality of hollow microneedles in a hollow microneedle array can have a cylindrical shape. In some embodiments, one or more of the plurality of hollow microneedles in a hollow microneedle array can have a square pyramidal shape. In some embodiments, one or more of the plurality of hollow microneedles in a hollow microneedle array can have a triangular pyramidal shape. In some embodiments, one or more of the plurality of hollow microneedles in a hollow microneedle array can have the shape of a hypodermic needle. In a preferred embodiment, the plurality of hollow microneedles in a hollow microneedle array each have the shape of a conventional hypodermic needle.

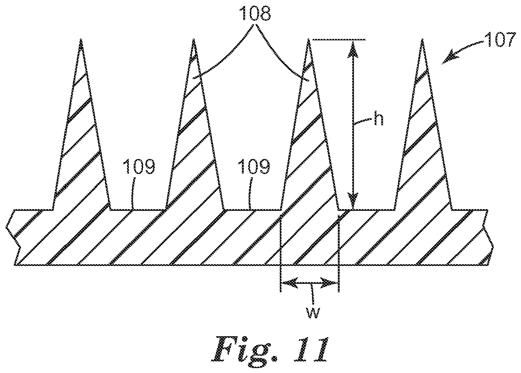

FIG. 11 shows a portion of the microneedle array 107 that includes four microneedles 108 (of which two are referenced in FIG.11) positioned on the microneedle array substrate 109. Each microneedle 108 has a height h, which is the length from the tip of the microneedle 108 to the microneedle base at substrate 109. Either the height of a single microneedle or the average height of all microneedles on the microneedle array can be referred to as the height of the microneedle, h. In some embodiments, each of the plurality of microneedles (or the average of all of the plurality of microneedles) has a height of about 100 to about 3000 micrometers, in some embodiments, about 100 to about 1500 micrometers, in some embodiments, about 100 to about1200 micrometers, and, in some embodiments, about 100 to about 1000 micrometers.

In some embodiments, each of the plurality of microneedles (or the average of all of the plurality of microneedles) has a height of about 200 to about 1200 micrometers, about 200 to about 1000 micrometers, about 200 to about 750 micrometers, or about 200 to about 600 micrometers.

In some embodiments, each of the plurality of microneedles (or the average of all of the plurality of microneedles) has a height of about 250 to about 1500 micrometers, about 500 to about 1000 micrometers, or about 500 to about 750 micrometers.

In some embodiments, each of the plurality of microneedles (or the average of all of the plurality of microneedles) has a height of about 800 to about 1400 micrometers.

In some embodiments, each of the plurality of microneedles (or the average of all of the plurality of microneedles) has a height of about 500.

In some embodiments, each of the plurality of microneedles (or the average of all of the plurality of microneedles) has a height of less than about 3000 micrometers. In other embodiments, each of the plurality of microneedles (or the average of all of the plurality of microneedles) has a height of less than about 1500 micrometers. In still other embodiments, each of the plurality of microneedles (or the average of all of the plurality of microneedles) has a height of less than about 1200 micrometers. In yet still other embodiments, each of the plurality of microneedles (or the average of all of the plurality of microneedles) has a height of less than about 1000 micrometers. In further embodiments, each of the plurality of microneedles (or the average of all of the plurality of microneedles) has a height of less than about 750 micrometers. In still further embodiments, each of the plurality of microneedles (or the average of all of the plurality of microneedles) has a height of less than about 600 micrometers.

In some embodiments, each of the plurality of microneedles (or the average of all of the plurality of microneedles) has a height of at least about 100 micrometers. In other embodiments, each of the plurality of microneedles (or the average of all of the plurality of microneedles) has a height of at least about 200 micrometers. In still other embodiments, each of the plurality of microneedles (or the average of all of the plurality of microneedles) has a height of at least about 250 micrometers. In further embodiments, each of the plurality of microneedles (or the average of all of the plurality of microneedles) has a height of at least about 500 micrometers. In still further embodiments, each of the plurality of microneedles (or the average of all of the plurality of microneedles) has a height of at least about 800 micrometers.

A single microneedle or the plurality of microneedles in a microneedle array can also be characterized by their aspect ratio. The aspect ratio of a microneedle is the ratio of the height of the microneedle, h to the width (at the base of the microneedle), w (as shown in FIG. 12). The aspect ratio can be presented as h:w. In some embodiments, each of the plurality of microneedles (or the average of all the plurality of microneedles) has (have) an aspect ratio in the range of 2:1 to 5:1. In some of these embodiments, each of the plurality of microneedles (or the average of all of the plurality of microneedles) has (have) an aspect ratio of at least 3:1.

In some embodiments, the array of microneedles contains about 100 to about 1500 microneedles per cm² of the array of microneedles.

In some embodiments, the array of microneedles contains about 200 to about 500 microneedles per cm² of the array of microneedles.

In some embodiments, the array of microneedles contains about 300 to about 400 microneedles per cm² of the array of microneedles.

In some embodiments, the array of microneedles contains about 3 to about 30 microneedles per cm² of the array of microneedles.

In some embodiments, the array of microneedles contains about 3 to about 20 microneedles per cm² of the array of microneedles.

In some embodiments, the array of microneedles contains about 10 to about 20 microneedles per cm² of the array of microneedles.

In some embodiments, each of the plurality of microneedles (or the average of all of the plurality of microneedles) in a microneedle array can penetrate into the skin to a depth of about 50 to about 1500 micrometers, about 50 to about 400 micrometers, or about 50 to about 250 micrometers.

In some embodiments, each of the plurality of microneedles (or the average of all of the plurality of microneedles) in a microneedle array can penetrate into the skin to a depth of about 100 to about 400 micrometers, or about 100 to about 300 micrometers.

In some embodiments, each of the plurality of microneedles (or the average of all of the plurality of microneedles) in a microneedle array can penetrate into the skin to a depth of about 150 to about 1500 micrometers, or about 800 to about 1500 micrometers.

In some embodiments, each of the plurality of microneedles (or the average of all of the plurality of microneedles) in a microneedle array can penetrate into the skin to a depth of about 400 to about 800 micrometers.

The microneedles 108 can be arranged in any desired pattern or distributed over the microneedle substrate 109 randomly. As shown in FIG. 1, in some embodiments, the microneedles 108 can be arranged in uniformly spaced rows. When arranged in rows, the rows can be arranged so that the microneedles 108 are aligned or offset. In some embodiments (not shown), the microneedles 108 can be arranged in a polygonal pattern such as a triangle, square, rectangle, pentagon, hexagon, heptagon, octagon, or trapezoid. In other embodiments (not shown), the microneedles 108 can be arranged in a circular or oval pattern.

In some embodiments, the surface area of the substrate 109 covered with microneedles 108 is about 0.1 cm² to about 20 cm². In some of these embodiments, the surface area of the substrate 109 covered with microneedles 108 is about 0.5 cm² to about 5 cm². In some other of these embodiments, the surface area of the substrate 109 covered with microneedles 108 is about 1 cm² to about 3 cm². In still other of these embodiments, the surface area of the substrate 109 covered with microneedles 108 is about 1 cm² to about 2 cm².

In some embodiments, the microneedles of the present disclosure can be disposed over substantially the entire surface of the array. In other embodiments (e.g., as shown in FIG. 1), a portion of the substrate 109 may not be provided with microneedles (that is, a portion of the substrate is non-structured). In some of these embodiments, the non-structured surface has an area of more than about 1 percent and less than about 75 percent of the total area of the device surface that faces the skin surface. In another of these embodiments, the non-structured surface has an area of more than about 0.65 cm² (0.10 square inch) to less than about 6.5 cm² (1 square inch).

In some embodiments of microneedle arrays, the average spacing between adjacent microneedles (as measured from microneedle tip to microneedle tip) is between about 200 micrometers and about 2000 micrometers. In other embodiments of microneedle arrays, the average spacing between adjacent microneedles is between about 200 micrometers and about 600 micrometers. In still other embodiments of microneedle arrays, the average spacing between adjacent microneedles is between about 200 micrometers and about 300 micrometers. In yet still other embodiments of microneedle arrays, the average spacing between adjacent microneedles is between about 500 micrometers and about 600 micrometers.

In some embodiments of microneedle arrays, the average spacing between adjacent microneedles (as measured from microneedle tip to microneedle tip) is greater than about 200 micrometers. In other embodiments of microneedle arrays, the average spacing between adjacent microneedles is greater than about 500 micrometers.

In some embodiments of microneedle arrays, the average spacing between adjacent microneedles is less than about 2000 micrometers. In other embodiments of microneedle arrays, the average spacing between adjacent microneedles is less than about 1000 micrometers. In still other embodiments of microneedle arrays, the average spacing between adjacent microneedles is less than about 600 micrometers. In yet still other embodiments of microneedle arrays, the average spacing between adjacent microneedles is less than about 300 micrometers.

The microneedle arrays can be manufactured in any suitable way such as by injection molding, compression molding, metal injection molding, stamping, photolithography, or extrusion.

The following embodiments are intended to be illustrative of the present disclosure and not limiting.

### EMBODIMENTS

### COUNTER ASSEMBLY EMBODIMENTS

1. A microneedle applicator comprising:
   a housing having a base and an opening formed in the base, wherein the base of the housing is configured to be positioned on a skin surface;
   a microneedle array holder configured to hold a microneedle array;
   an actuator movable with respect to the housing between a first position and a second position to cause the microneedle array holder to move, respectively, between
      a retracted position in which the microneedle array is recessed within the housing such that the microneedle array does not contact the skin surface when the base of the housing is positioned on the skin surface and the microneedle array is coupled to the microneedle array holder, and
      an extended position in which at least a portion of the microneedle array is positioned to contact the skin surface when the base of the housing is positioned on the skin surface and the microneedle array is coupled to the microneedle array holder;
   a first biasing element configured to bias the actuator in the first position; and
   a counter assembly configured to count a number of times the microneedle array holder is moved between the retracted position and the extended position.
2. The microneedle applicator of embodiment 1, wherein a microneedle array is coupled to the microneedle array holder, and wherein the counter assembly is configured to count a number of times the microneedle array contacts a skin surface.
3. The microneedle applicator of embodiment 1 or 2, wherein the actuator is movable against a bias of the first biasing element to move from the first position to the second position to move the microneedle array holder from the retracted position to the extended position.
4. The microneedle applicator of any of embodiments 1-3, wherein the counter assembly is mechanically coupled to the actuator and the first biasing element.
5. The microneedle applicator of any of embodiments 1-4, wherein the counter mechanism is driven by the actuator and the first biasing element.
6. The microneedle applicator of any of embodiments 1-5, wherein the actuator is movable between the first position and the second position to cause the counter assembly to increment a count.
7. The microneedle applicator of any of embodiments 1-6, wherein the counter assembly includes a counter, and wherein the counter includes a display configured to display a count representative of a number of times the microneedle array holder is moved to the extended position.
8. The microneedle applicator of embodiment 7, wherein at least a portion of the counter is rotatably movable relative to the housing.
9. The microneedle applicator of embodiment 7 or 8, wherein the housing includes a longitudinal axis, and wherein at least a portion of the counter is rotatably movable relative to the longitudinal axis.
10. The microneedle applicator of embodiment 9, wherein the counter is discretely rotatable relative to the longitudinal axis of the housing.
11. The microneedle applicator of any of embodiments 7-10, wherein the housing includes an opening configured to display the count, and wherein at least a portion of the counter is rotatably movable relative to the opening in the housing.
12. The microneedle applicator of any of embodiments 7-11, wherein the counter assembly further includes a guide, wherein at least a portion of the counter is rotatably and slidably movable relative to the guide.
13. The microneedle applicator of embodiment 12, wherein the counter includes a first cam surface and the guide includes a second cam surface, and wherein the first cam surface and the second cam surface are configured to cam along one another to cause at least a portion of the counter to rotate relative to the guide.
14. The microneedle applicator of embodiment 12 or 13, wherein the guide is fixed relative to the housing.
15. The microneedle applicator of any of embodiments 12-14, wherein the counter includes a first portion and a second portion, wherein the first portion and the second portion of the counter are each slidably movable relative to the guide, wherein the second portion of the counter includes the display, and wherein the second portion is further rotatably movable relative to the guide.
16. The microneedle applicator of embodiment 15, wherein the second portion of the counter is further rotatably movable relative to the first portion of the counter.
17. The microneedle applicator of embodiment 15 or 16, wherein the guide includes a plurality of channels, and wherein at least one of the first portion and the second portion of the counter include at least one projection, each projection dimensioned to be received in one of the plurality of channels.
18. The microneedle applicator of embodiment 17, wherein the plurality of channels and the at least one projection allow the counter to be slid relative to the guide.
19. The microneedle applicator of any of embodiments 15-18, wherein the first portion of the counter includes a plurality of first engagement features, and wherein the second portion of the counter includes a plurality of second engagement features configured to engage the plurality of first engagement features.
20. The microneedle applicator of embodiment 19, wherein the plurality of second engagement features include a first cam surface, wherein the guide includes a second cam surface, and wherein the first cam surface and the second cam surface are configured to cam along one another to cause the second portion of the counter to rotate relative to the guide.
21. The microneedle applicator of any of embodiments 1-20, wherein the counter assembly includes a first cam surface and a second cam surface configured to cam along one another to cause at least a portion of the counter assembly to rotate to increment a count.
22. The microneedle applicator of any of embodiments 1-21, wherein the microneedle array holder is movable along an actuation axis between its retracted position and its extended position, wherein the housing includes a cover, and wherein the cover provides the base of the housing and the opening formed in the base, wherein the cover is movable between
   a first position in which the actuation axis passes through the opening in the base of the cover, and
   a second position in which the cover is located in an off-axis position in which the actuation axis does not pass through the opening in the base of the cover.
23. The microneedle applicator of embodiment 22, wherein, when the cover is in its second position, the microneedle array holder is movable to the extended position to load a microneedle array.
24. The microneedle applicator of embodiment 22 or 23, wherein the microneedle array holder is recessed within the microneedle applicator when the cover is in the first position, and wherein the microneedle array holder is exposed when the cover is in the second position.
25. The microneedle applicator of any of embodiments 1-24, further comprising a second biasing element configured to bias the microneedle array holder in the extended position, wherein the microneedle array holder is movable against the bias of the second biasing element, when a threshold application force is applied, to move the microneedle array holder from the extended position to a dampened position.

### FORCE-DAMPENING EMBODIMENTS

1. A microneedle applicator comprising:
   a housing having a base and an opening formed in the base, wherein the base of the housing is configured to be positioned on a skin surface;
   a microneedle array holder configured to hold a microneedle array;
   an actuator movable with respect to the housing between a first position and a second position to cause the microneedle array holder to move, respectively, between
      a retracted position in which the microneedle array is recessed within the housing such that the microneedle array does not contact the skin surface when the base of the housing is positioned on the skin surface and the microneedle array is coupled to the microneedle array holder, and
      an extended position in which at least a portion of the microneedle array is positioned to contact the skin surface when the base of the housing is positioned on the skin surface and the microneedle array is coupled to the microneedle array holder;
      wherein, after the actuator has been moved to the second position, the microneedle array holder is further movable between the extended position and a dampened position, wherein the dampened position is located between the retracted position and the extended position, such that the microneedle array holder is located within the housing when in the dampened position;
   a first biasing element configured to bias the actuator in the first position; and
   a second biasing element to bias the microneedle array holder in the extended position, wherein the microneedle array holder is movable against the bias of the second biasing element, when a threshold application force is applied, to move the microneedle array holder from the extended position to the dampened position.
2. The microneedle applicator of embodiment 1, wherein the microneedle array holder has a first side configured to be coupled to the microneedle array and configured to be positioned toward the skin surface, and wherein the microneedle array holder is movable against the bias of the second biasing element when a threshold application force is applied to the first side of the microneedle array holder.
3. The microneedle applicator of embodiment 1 or 2, wherein the base of the housing is in contact with the skin surface when the microneedle array holder is in the extended position and the dampened position.
4. The microneedle applicator of any of embodiments 1-3, wherein when the microneedle array holder is in the dampened position, at least a portion of the microneedle array is positioned to contact the skin surface when the base of the housing is positioned on the skin surface.
5. The microneedle applicator of any of embodiments 1-4, wherein the actuator is movable against a bias of the first biasing element to move from the first position to the second position to move the microneedle array holder from the retracted position to the extended position.
6. The microneedle applicator of any of embodiments 1-5, wherein the first biasing element is a spring.
7. The microneedle applicator of any of embodiments 1-6, wherein the first biasing element is compressed when the actuator is in the second position.
8. The microneedle applicator of any of embodiments 1-7, wherein the second biasing element is a spring.
9. The microneedle applicator of any of embodiments 1-8, wherein the second biasing element is compressed when the microneedle array holder is in the dampened position.
10. The microneedle applicator of any of embodiments 1-9, wherein the second biasing element is compressed between the microneedle array holder and the actuator when the microneedle array holder is in the dampened position.
11. The microneedle applicator of any of embodiments 1-10, wherein the first biasing element biases the actuator upwardly relative to the skin surface, and wherein the second biasing element biases the microneedle array holder downwardly relative to the skin surface.
12. The microneedle applicator of any of embodiments 1-11, wherein the actuator is movable from the first position to the second position in a first direction, wherein the microneedle array holder is movable from the extended position to the dampened position in a second direction, and wherein the first direction is different from the second direction.
13. The microneedle applicator of embodiment 12, wherein the first direction and the second direction are opposite one another.
14. The microneedle applicator of any of embodiments 1-13, wherein the microneedle array holder is movable between the extended position and the dampened position when the actuator is maintained in the second position.
15. The microneedle applicator of any of embodiments 1-14, wherein the actuator includes a plunger, and wherein the first biasing element is compressed between the plunger and a wall of the housing when the plunger is in the second position.
16. The microneedle applicator of any of embodiments 1-15, wherein the actuator includes a plunger, and wherein the second biasing element is compressed between the microneedle array holder and the plunger when the microneedle array holder is in the dampened position.
17. The microneedle applicator of any of embodiments 1-16, wherein the actuator includes a plunger, and wherein at least a portion of the plunger is configured to be coupled to the microneedle array holder.
18. The microneedle applicator of any of embodiments 1-17, wherein the microneedle array holder is movable along an actuation axis between its retracted position and its extended position, wherein the housing includes a cover, and wherein the cover provides the base of the housing and the opening formed in the base, wherein the cover is movable between
   a first position in which the actuation axis passes through the opening in the base of the cover, and
   a second position in which the cover is located in an off-axis position in which the actuation axis does not pass through the opening in the base of the cover.
19. The microneedle applicator of embodiment 18, wherein, when the cover is in its second position, the microneedle array holder is movable to the extended position to load a microneedle array.
20. The microneedle applicator of embodiment 18 or 19, wherein the microneedle array holder is recessed within the microneedle applicator when the cover is in the first position, and wherein the microneedle array holder is exposed when the cover is in the second position.
21. The microneedle applicator of any of embodiments 1-20, further comprising a counter assembly configured to count a number of times the microneedle array holder is moved between the retracted position and the extended position.

### MOVABLE COVER EMBODIMENTS

1. A microneedle applicator comprising:
   a microneedle array holder configured to hold a microneedle array, wherein the microneedle array holder is movable relative to a skin surface along an actuation axis between a retracted position and an extended position;
   a housing configured to at least partially house the microneedle array holder at least when the microneedle array holder is in the retracted position;
   a cover coupled to the housing, the cover having a base and an opening formed in the base, wherein the base of the cover is configured to be positioned on the skin surface, wherein the cover is movable between
      a first position in which the actuation axis passes through the opening in the base of the cover, and
      a second position in which the cover is located in an off-axis position in which the actuation axis does not pass through the opening in the base of the cover.
2. The microneedle applicator of embodiment 1, wherein the cover is removable from the housing.
3. The microneedle applicator of embodiment 1 or 2, wherein the cover is removable from the housing when the cover is in the first position and when the cover is in the second position.
4. The microneedle applicator of any of embodiments 1-3, wherein the cover is decoupled from the housing when the cover is in the second position.
5. The microneedle applicator of any of embodiments 1-4, wherein the housing is elongated along a longitudinal axis, and wherein the actuation axis is parallel to the longitudinal axis of the housing.
6. The microneedle applicator of embodiment 5, wherein the longitudinal axis of the housing passes through the opening in the base of the cover when the cover is in the first position.
7. The microneedle applicator of any of embodiments 1-6, wherein the microneedle array holder is recessed within the microneedle applicator when the cover is in the first position, and wherein the microneedle array holder is exposed when the cover is in the second position.
8. The microneedle applicator of any of embodiments 1-7, wherein the microneedle array holder is recessed within a cavity defined by the housing and the cover when the cover is in the first position, and wherein the microneedle array holder is exposed when the cover is in the second position.
9. The microneedle applicator of any of embodiments 1-8, wherein the cover is coupled to the housing when the cover is in the first position and the second position.
10. The microneedle applicator of any of embodiments 1-9, wherein the housing includes a base, and wherein the cover is coupled to and covers the base of the housing when the cover is in the first position, but wherein the base of the housing is exposed when the cover is in the second position.
11. The microneedle applicator of embodiment 10, wherein at least a portion of the microneedle array holder extends beyond the base of the housing when the microneedle array holder is in its extended position.
12. The microneedle applicator of embodiment 10 or 11, wherein the base of the housing includes an opening formed therein, and wherein the microneedle array holder is movable through the opening formed in the base of the housing when the microneedle array holder moves between its retracted position and its extended position.
13. The microneedle applicator of any of embodiments 10-12, wherein the housing includes a cavity that extends through the base to define an opening in the base, and wherein the microneedle array holder is at least partially located in the cavity of the housing when the microneedle array holder is in its retracted position, and wherein the microneedle array holder extends through the opening and beyond the base of the housing when the microneedle array holder is in its extended position.
14. The microneedle applicator of any of embodiments 1-13, wherein the cover includes a plurality of second positions, and wherein the cover is movable between the first position and any of the plurality of second positions.
15. The microneedle applicator of any of embodiments 1-14, wherein the cover is pivotally movable with respect to the housing.
16. The microneedle applicator of any of embodiments 1-15, wherein the cover is slidably movable with respect to the housing.
17. The microneedle applicator of any of embodiments 1-16, wherein the cover is pivotally and slidably movable with respect to the housing.
18. The microneedle applicator of any of embodiments 1-17, wherein the cover is configured to pivot about a rotational axis that is oriented substantially perpendicularly with respect to a longitudinal axis of the housing.
19. The microneedle applicator of any of embodiments 1-18, wherein the cover is configured to pivot about a rotational axis that is oriented substantially perpendicularly with respect to the actuation axis.
20. The microneedle applicator of any of embodiments 1-19, wherein the cover is configured to pivot about a rotational axis that is oriented substantially perpendicularly with respect to a direction that is normal to the skin surface.
21. The microneedle applicator of any of embodiments 1-20, wherein the cover is configured to pivot about a rotational axis that is oriented substantially parallel with respect to the skin surface.
22. The microneedle applicator of any of embodiments 1-21, wherein the cover and the housing are coupled together by a snap-fit-type engagement when the cover is in the first position.
23. The microneedle applicator of any of embodiments 1-22, wherein the second position is a discrete position defined by a detent.
24. The microneedle applicator of any of embodiments 1-23, wherein the cover is further movable between the first position, the second position, and a third position spaced a distance along the actuation axis from the first position.
25. The microneedle applicator of embodiment 24, wherein when the cover is in the first position, the microneedle applicator is in an assembled configuration, and wherein when the cover is in the third position, the microneedle applicator is in a disassembled configuration.
26. The microneedle applicator of embodiment 24 or 25, wherein the actuation axis passes through the opening in the base of the cover when the cover is in the third position.
27. The microneedle applicator of any of embodiments 24-26, wherein the third position is located intermediately of the first position and the second position, such that the cover moves through the third position when moved between the first position and the second position.
28. The microneedle applicator of any of embodiments 24-27, wherein the cover is coupled to the housing when the cover is in the first position, the second position, and the third position.
29. The microneedle applicator of any of embodiments 24-28, wherein the housing includes a base, and wherein the cover is coupled to and covers the base of the housing when the cover is in the first position, but wherein the base of the housing is exposed when the cover is in the second position and the third position.
30. The microneedle applicator of embodiment 29, wherein at least a portion of the microneedle array holder extends beyond the base of the housing when the microneedle array holder is in its extended position.
31. The microneedle applicator of embodiment 29 or 30, wherein the base of the housing includes an opening formed therein, and wherein the microneedle array is movable through the opening formed in the base of the housing when the microneedle array moves between its retracted position and its extended position.
32. The microneedle applicator of any of embodiments 24-31, wherein the cover is pivotally movable between the third position and the second position.
33. The microneedle applicator of any of embodiments 24-32, wherein the cover is slidably movable between the first position and the third position.
34. The microneedle applicator of any of embodiments 24-33, wherein the cover includes a plurality of second positions, and wherein the cover is movable between the third position and any of the plurality of second positions.
35. The microneedle applicator of any of embodiments 24-34, wherein the cover is free to pivot about an axis when the cover is in the third position.
36. The microneedle applicator of embodiment 35, wherein the cover is movable from the third position to the second position by pivoting about the axis.
37. The microneedle applicator of any of embodiments 24-36, wherein the second position is a discrete position defined by a detent.
38. The microneedle applicator of any of embodiments 24-37, wherein the cover is slidable along a longitudinal axis of the housing to move between the first position and the third position, and wherein the cover is rotatable about an axis that is oriented substantially perpendicularly with respect to the longitudinal axis of the housing to move between the third position and the second position.
39. The microneedle applicator of any of embodiments 1-38, wherein when the cover is in the first position and the microneedle array is coupled to the microneedle array holder, the microneedle array is movable between
   a first position corresponding to the retracted position of the microneedle array holder in which the microneedle array is recessed with respect to the base of the cover, such that the microneedle array does not extend beyond the base of the cover, and
   a second position corresponding to the extended position of the microneedle array holder in which at least a portion of the microneedle array is positioned to contact the skin surface when the base of the housing is positioned on the skin surface.
40. The microneedle applicator of any of embodiments 1-39, wherein when the cover is in the second position, the microneedle array holder is movable between its retracted position and its extended position to couple a microneedle array to the microneedle array holder.
41. The microneedle applicator of any of embodiments 1-40, wherein the microneedle array holder is biased in its retracted position by a biasing element.
42. The microneedle applicator of any of embodiments 1-41, further comprising an actuator movable with respect to the housing between a first position and a second position to cause the microneedle array holder to move between the retracted position and the extended position.
43. The microneedle applicator of embodiment 42, further comprising a first biasing element configured to bias the actuator in the first position.
44. The microneedle application of embodiment 43, wherein the actuator is movable against a bias of the first biasing element to move from the first position to the second position to move the microneedle array holder from the retracted position to the extended position.
45. The microneedle application of embodiment 43 or 44, further comprising a second biasing element configured to bias the microneedle array holder in the extended position, wherein the microneedle array holder is movable against the bias of the second biasing element, when a threshold application force is applied, to move the microneedle array holder from the extended position to a dampened position.
46. The microneedle applicator of any of embodiments 1-45, further comprising a counter assembly configured to count a number of times the microneedle array holder is moved between the retracted position and the extended position.
47. The microneedle applicator of any of embodiments 1-43, wherein when the microneedle array holder is in the retracted position and the microneedle array is coupled to the microneedle array holder, the microneedle array is recessed within the housing such that the microneedle array does not contact the skin surface when the base of the cover is positioned in contact with the skin surface, and wherein when the microneedle array holder is in the extended position and the microneedle array is coupled to the microneedle array holder, at least a portion of the microneedle array is positioned to contact the skin surface when the base of the cover is in contact with the skin surface.
48. A kit comprising:
   the microneedle applicator of any preceding embodiment; and
   a tray comprising a plurality of wells, wherein each well includes a microneedle array.

The embodiments described above and illustrated in the figures are presented by way of example only and are not intended as a limitation upon the concepts and principles of the present disclosure. As such, it will be appreciated by one having ordinary skill in the art that various changes in the elements and their configuration and arrangement are possible without departing from the scope of the present disclosure.

Various features and aspects of the present disclosure are set forth in the following claims.

## Claims

1. A microneedle applicator (100) comprising:
a housing (102) having a base (112) and an opening formed in the base (112), wherein the base (112) of the housing (102) is configured to be positioned on a skin surface;
a microneedle array holder (106) configured to hold a microneedle array (107);
an actuator (104) movable with respect to the housing (102) between a first position and a second position to cause the microneedle array holder (106) to move, respectively, between
a retracted position in which the microneedle array (107) is recessed within the housing (102) such that the microneedle array (107) does not contact the skin surface when the base (112) of the housing (102) is positioned on the skin surface and the microneedle array (107) is coupled to the microneedle array holder, and
an extended position in which at least a portion of the microneedle array (107) is positioned to contact the skin surface when the base (112) of the housing (102) is positioned on the skin surface and the microneedle array (107) is coupled to the microneedle array holder (106);
a first biasing element (111) configured to bias the actuator (104) in the first position; and
a counter assembly (110) configured to count a number of times the microneedle array holder (107) is moved between the retracted position and the extended position.

2. The microneedle applicator (100) of claim 1, wherein a microneedle array (107) is coupled to the microneedle array holder (106), and wherein the counter assembly (110) is configured to count a number of times the microneedle array (107) contacts a skin surface.

3. The microneedle applicator (100) of claim 1 or 2, wherein the actuator (104) is movable against a bias of the first biasing element (111) to move from the first position to the second position to move the microneedle array holder (106) from the retracted position to the extended position.

4. The microneedle applicator (100) of any of claims 1-3, wherein the counter assembly (110) is mechanically coupled to the actuator (104) and the first biasing element (111).

5. The microneedle applicator (100) of any of claims 1-4, wherein the counter mechanism is driven by the actuator (104) and the first biasing element (111).

6. The microneedle applicator (100) of any of claims 1-5, wherein the actuator (104) is movable between the first position and the second position to cause the counter assembly (110) to increment a count.

7. The microneedle applicator (100) of any of claims 1-6, wherein the counter assembly (110) includes a counter (188), and wherein the counter (188) includes a display (168) configured to display a count representative of a number of times the microneedle array holder (106) is moved to the extended position.

8. The microneedle applicator (100) of claim 7, wherein at least a portion of the counter (188) is rotatably movable relative to the housing (102).

9. The microneedle applicator (100) of any of claims 7-8, wherein the housing (102) includes an opening configured to display the count, and wherein at least a portion of the counter (188) is rotatably movable relative to the opening in the housing (102).

10. The microneedle applicator (100) of any of claims 7-9, wherein the counter assembly (110) further includes a guide (186), wherein at least a portion of the counter (188) is rotatably and slidably movable relative to the guide (186).

11. The microneedle applicator (100) of claim 10, wherein the counter (188) includes a first cam surface and the guide (186) includes a second cam surface, and wherein the first cam surface and the second cam surface are configured to cam along one another to cause at least a portion of the counter (188) to rotate relative to the guide (186).

12. The microneedle applicator (100) of claim 10 or 11, wherein the guide (186) is fixed relative to the housing (102).

13. The microneedle applicator (100) of any of claims 10-12, wherein the counter (188) includes a first portion (187) and a second portion (189), wherein the first portion (187) and the second portion (189) of the counter (188) are each slidably movable relative to the guide (186), wherein the second portion of the counter (188) includes the display, and wherein the second portion (189) is further rotatably movable relative to the guide (186).

14. The microneedle applicator (100) of claim 13, wherein the second portion (189) of the counter (188) is further rotatably movable relative to the first portion (187) of the counter (188).

15. The microneedle applicator (100) of claim 13 or 14, wherein the guide (186) includes a plurality of channels (149), and wherein at least one of the first portion (187) and the second portion (189) of the counter (188) include at least one projection (160), each projection (160) dimensioned to be received in one of the plurality of channels (149).

## Patentansprüche

1. Mikronadelapplikator (100), aufweisend:
ein Gehäuse (102) mit einer Basis (112) und einer Öffnung, die in der Basis (112) gebildet ist, wobei die Basis (112) des Gehäuses (102) dazu konfiguriert ist, auf einer Hautoberfläche positioniert zu werden;
einen Mikronadelanordnungshalter (106), der dazu konfiguriert ist, eine Mikronadelanordnung (107) zu halten;
ein Betätigungselement (104), das in Bezug auf das Gehäuse (102) zwischen einer ersten Position und einer zweiten Position bewegbar ist, um zu bewirken, dass sich der Mikronadelanordnungshalter (106) jeweils bewegt zwischen
einer zurückgezogenen Position, in der die Mikronadelanordnung (107) innerhalb des Gehäuses (102) so zurückgesetzt ist, dass die Mikronadelanordnung (107) die Hautoberfläche nicht berührt, wenn die Basis (112) des Gehäuses (102) auf der Hautoberfläche positioniert ist und die Mikronadelanordnung (107) mit dem Mikronadelanordnungshalter gekoppelt ist, und
einer ausgefahrenen Position, in der mindestens ein Abschnitt der Mikronadelanordnung (107) so positioniert ist, dass sie die Hautoberfläche berührt, wenn die Basis (112) des Gehäuses (102) auf der Hautoberfläche positioniert und die Mikronadelanordnung (107) mit dem Mikronadelanordnungshalter (106) gekoppelt ist;
ein erstes Vorspannelement (111), das dazu konfiguriert ist, das Betätigungselement (104) in der ersten Position vorzuspannen; und
eine Zähleranordnung (110), die dazu konfiguriert ist, die Anzahl an Malen zu zählen, die der Mikronadelanordnungshalter (107) zwischen der zurückgezogenen Position und der ausgefahrenen Position hin und her bewegt wird.

2. Mikronadelapplikator (100) nach Anspruch 1, wobei eine Mikronadelanordnung (107) mit dem Mikronadelanordnungshalter (106) gekoppelt ist und wobei die Zähleranordnung (110) dazu konfiguriert ist, die Anzahl an Malen zu zählen, die die Mikronadelanordnung (107) eine Hautoberfläche berührt.

3. Mikronadelapplikator (100) nach Anspruch 1 oder 2, wobei das Betätigungselement (104) gegen eine Vorspannung des ersten Vorspannelements (111) bewegbar ist, um sich von der ersten Position zu der zweiten Position zu bewegen, um den Mikronadelanordnungshalter (106) aus der zurückgezogenen Position in die ausgefahrene Position zu bewegen.

4. Mikronadelapplikator (100) nach einem der Ansprüche 1-3, wobei die Zähleranordnung (110) mechanisch mit dem Betätigungselement (104) und dem ersten Vorspannelement (111) gekoppelt ist.

5. Mikronadelapplikator (100) nach einem der Ansprüche 1-4, wobei der Zählmechanismus durch das Betätigungselement (104) und das erste Vorspannelement (111) angetrieben wird.

6. Mikronadelapplikator (100) nach einem der Ansprüche 1-5, wobei das Betätigungselement (104) zwischen der ersten Position und der zweiten Position bewegbar ist, um zu bewirken, dass die Zähleranordnung (110) einen Zählwert inkrementiert.

7. Mikronadelapplikator (100) nach einem der Ansprüche 1-6, wobei die Zähleranordnung (110) einen Zähler (188) umfasst und wobei der Zähler (188) eine Anzeige (168) umfasst, die dazu konfiguriert ist, einen Zählwert anzuzeigen, der die Anzahl an Malen darstellt, die der Mikronadelanordnungshalter (106) in die ausgefahrene Position bewegt wird.

8. Mikronadelapplikator (100) nach Anspruch 7, wobei wenigstens ein Abschnitt des Zählers (188) in Bezug auf das Gehäuse (102) drehbar bewegbar ist.

9. Mikronadelapplikator (100) nach einem der Ansprüche 7-8, wobei das Gehäuse (102) eine Öffnung aufweist, die dazu konfiguriert ist, den Zählwert anzuzeigen, und wobei mindestens ein Abschnitt des Zählers (188) in Bezug auf die Öffnung in dem Gehäuse (102) drehbar bewegbar ist.

10. Mikronadelapplikator (100) nach einem der Ansprüche 7-9, wobei die Zähleranordnung (110) ferner eine Führung (186) umfasst, wobei mindestens ein Abschnitt des Zählers (188) in Bezug auf die Führung (186) drehbar und verschiebbar bewegbar ist.

11. Mikronadelapplikator (100) nach Anspruch 10, wobei der Zähler (188) eine erste Nockenfläche aufweist und die Führung (186) eine zweite Nockenfläche aufweist und wobei die erste Nockenfläche und die zweite Nockenfläche dazu konfiguriert sind, sich aneinander entlang zu bewegen, um zu bewirken, dass sich zumindest ein Abschnitt des Zählers (188) in Bezug auf die Führung (186) dreht.

12. Mikronadelapplikator (100) nach Anspruch 10 oder 11, wobei die Führung (186) in Bezug auf das Gehäuse (102) fixiert ist.

13. Mikronadelapplikator (100) nach einem der Ansprüche 10-12, wobei der Zähler (188) einen ersten Abschnitt (187) und einen zweiten Abschnitt (189) aufweist, wobei der erste Abschnitt (187) und der zweite Abschnitt (189) des Zählers (188) jeweils in Bezug auf die Führung (186) verschiebbar bewegbar ist, wobei der zweite Abschnitt des Zählers (188) die Anzeige aufweist und wobei der zweite Abschnitt (189) ferner in Bezug auf die Führung (186) drehbar bewegbar ist.

14. Mikronadelapplikator (100) nach Anspruch 13, wobei der zweite Abschnitt (189) des Zählers (188) ferner in Bezug auf den ersten Abschnitt (187) des Zählers (188) drehbar bewegbar ist.

15. Mikronadelapplikator (100) nach Anspruch 13 oder 14, wobei die Führung (186) mehrere Kanäle (149) aufweist und wobei der erste Abschnitt (187) und/oder der zweite Abschnitt (189) des Zählers (188) mindestens einen Vorsprung (160) aufweist, wobei jeder Vorsprung (160) so bemessen ist, um in einem der Vielzahl von Kanälen (149) aufgenommen zu werden.

## Revendications

1. Applicateur à micro-aiguilles (100) comprenant :
un logement (102) ayant une base (112) et une ouverture formée dans la base (112), dans lequel la base (112) du logement (102) est configurée pour être positionnée sur une surface de peau ;
un support de réseau de micro-aiguilles (106) configuré pour maintenir un réseau de micro-aiguilles (107) ;
un actionneur (104) mobile par rapport au logement (102) entre une première position et une deuxième position pour amener le support de réseau de micro-aiguilles (106) à se déplacer, respectivement, entre
une position rétractée dans laquelle le réseau de micro-aiguilles (107) est en retrait au sein du logement (102) de telle sorte que le réseau de micro-aiguilles (107) ne vient pas en contact avec la surface de peau lorsque la base (112) du logement (102) est positionnée sur la surface de la peau et que le réseau de micro-aiguilles (107) est couplé au support de réseau de micro-aiguilles, et
une position étendue dans laquelle au moins une partie du réseau de micro-aiguilles (107) est positionnée pour venir en contact avec la surface de la peau lorsque la base (112) du logement (102) est positionnée sur la surface de peau et que le réseau de micro-aiguilles (107) est couplé au support de réseau de micro-aiguilles (106) ;
un premier élément de sollicitation (111) configuré pour solliciter l'actionneur (104) dans la première position ; et
un ensemble compteur (110) configuré pour compter un nombre de fois que le support de réseau de micro-aiguilles (107) est déplacé entre la position rétractée et la position étendue.

2. Applicateur à micro-aiguilles (100) selon la revendication 1, dans lequel un réseau de micro-aiguilles (107) est couplé au support de réseau de micro-aiguilles (106), et dans lequel l'ensemble compteur (110) est configuré pour compter un nombre de fois que le réseau de micro-aiguilles (107) vient en contact avec une surface de peau.

3. Applicateur à micro-aiguilles (100) selon la revendication 1 ou 2, dans lequel l'actionneur (104) peut être déplacé à l'encontre d'une sollicitation du premier élément de sollicitation (111) pour se déplacer de la première position à la deuxième position pour déplacer le support de réseau de micro-aiguilles (106) de la position rétractée à la position étendue.

4. Applicateur à micro-aiguilles (100) selon l'une quelconque des revendications 1 à 3, dans lequel l'ensemble compteur (110) est couplé mécaniquement à l'actionneur (104) et au premier élément de sollicitation (111).

5. Applicateur à micro-aiguilles (100) selon l'une quelconque des revendications 1 à 4, dans lequel le mécanisme de compteur est entraîné par l'actionneur (104) et le premier élément de sollicitation (111).

6. Applicateur à micro-aiguilles (100) selon l'une quelconque des revendications 1 à 5, dans lequel l'actionneur (104) peut être déplacé entre la première position et la deuxième position pour amener l'ensemble compteur (110) à incrémenter un comptage.

7. Applicateur à micro-aiguilles (100) selon l'une quelconque des revendications 1 à 6, dans lequel l'ensemble compteur (110) inclut un compteur (188), et dans lequel le compteur (188) inclut un affichage (168) configuré pour afficher un comptage représentatif d'un nombre de fois que le support de réseau de micro-aiguilles (106) est déplacé à la position étendue.

8. Applicateur à micro-aiguilles (100) selon la revendication 7, dans lequel au moins une partie du compteur (188) peut se déplacer de manière rotative par rapport au logement (102).

9. Applicateur à micro-aiguilles (100) selon l'une quelconque des revendications 7 à 8, dans lequel le logement (102) inclut une ouverture configurée pour afficher le comptage, et dans lequel au moins une partie du compteur (188) peut se déplacer de manière rotative par rapport à l'ouverture dans le logement (102).

10. Applicateur à micro-aiguilles (100) selon l'une quelconque des revendications 7 à 9, dans lequel l'ensemble compteur (110) inclut en outre un guide (186), dans lequel au moins une partie du compteur (188) peut se déplacer de manière rotative et coulissante par rapport au guide (186).

11. Applicateur à micro-aiguilles (100) selon la revendication 10, dans lequel le compteur (188) inclut une première surface de came et le guide (186) inclut une deuxième surface de came, et dans lequel la première surface de came et la deuxième surface de came sont configurées pour servir de came le long l'une de l'autre pour amener au moins une partie du compteur (188) à tourner par rapport au guide (186).

12. Applicateur à micro-aiguilles (100) selon la revendication 10 ou 11, dans lequel le guide (186) est fixe par rapport au logement (102).

13. Applicateur à micro-aiguilles (100) selon l'une quelconque des revendications 10 à 12, dans lequel le compteur (188) inclut une première partie (187) et une deuxième partie (189), dans lequel la première partie (187) et la deuxième partie (189) du compteur (188) peuvent chacune se déplacer de manière coulissante par rapport au guide (186), dans lequel la deuxième partie du compteur (188) inclut l'affichage, et dans lequel la deuxième partie (189) peut en outre se déplacer de manière rotative par rapport au guide (186).

14. Applicateur à micro-aiguilles (100) selon la revendication 13, dans lequel la deuxième partie (189) du compteur (188) peut en outre se déplacer de manière rotative par rapport à la première partie (187) du compteur (188).

15. Applicateur à micro-aiguilles (100) selon la revendication 13 ou 14, dans lequel le guide (186) inclut une pluralité de canaux (149), et dans lequel au moins l'une parmi la première partie (187) et la deuxième partie (189) du compteur (188) inclut au moins une saillie (160), chaque saillie (160) étant dimensionnée pour être reçue dans l'un parmi la pluralité de canaux (149).
